(19) Europäisches Patentamt
European Patent Office
Office européen des brevets

(11) EP 4 036 081 A1

# (12) EUROPEAN PATENT APPLICATION

(43) Date of publication:
**03.08.2022 Bulletin 2022/31**

(21) Application number: **21305108.9**

(22) Date of filing: **28.01.2021**

(51) International Patent Classification (IPC):
***C07D 225/04*** *(2006.01)* ***C07D 245/04*** *(2006.01)*
***C07D 255/04*** *(2006.01)* ***C07D 498/22*** *(2006.01)*
***A61K 49/10*** *(2006.01)*

(52) Cooperative Patent Classification (CPC):
**C07D 498/22; A61K 49/1806; A61K 49/189; C07D 225/04; C07D 245/04; C07D 255/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Université de Caen Normandie**
**14000 Caen (FR)**

(72) Inventors:
- **DUBOST, Emmanuelle**
**14610 Thaon (FR)**
- **CAILLY, Thomas**
**14610 Thaon (FR)**
- **FABIS, Frédéric**
**14000 Caen (FR)**
- **VIGIER, Clément**
**14000 Caen (FR)**
- **MALZERT-FREON, Aurélie**
**14310 Amayé sur Seulles (FR)**
- **GROO, Anne-Claire**
**14930 Eterville (FR)**

(74) Representative: **Gevers & Orès**
**Immeuble le Palatin 2**
**3 Cours du Triangle**
**CS 80165**
**92939 Paris La Défense Cedex (FR)**

## (54) AZA-CRYPTOPHANES, PROCESSES FOR PREPARATION THEREOF, AND THEIR USES

(57) The present invention concerns aza-cryptophanes, processes for preparation thereof, and their uses, in particular as in vivo diagnostic tools when complexing a hyperpolarized noble element, or in nanoemulsions.

EP 4 036 081 A1

## Description

The present invention concerns aza-cryptophanes, processes for preparation thereof, and their uses, in particular as *in vivo* diagnostic tools when complexing a hyperpolarized noble element, or in nanoemulsions.

Cryptophanes are cage molecules capable of encapsulating atoms or small molecules. These molecules consist of two cyclotriveratrylene units linked together by ether bridges of different lengths. The use of these cage molecules is of particular interest in the field of MRI imaging, using hyperpolarized xenon (Xe-HP) as a contrast agent.

Indeed, xenon is a promising contrast agent in the field of molecular MRI imaging, thanks to its hyperpolarization and HyperCEST techniques, this atom being detected with high contrast and in very low quantities.

Cryptophanes are the best hosts for this gas described to date.

However, Xe-HP MRI imaging using this type of *in vivo* bioprobe is still lacking, in particular due to the poor solubility of this type of cage in biocompatible media.

In addition, as this gas is non-specific, it may be advantageously vectorized to selectively reach a given biological target.

But such a vectorization requires beforehand the synthesis of monofunctionalised cryptophane, in order to attach the linker to this latter. This type of precursor is obtained most of the time from the symmetrical cryptophane, via a step of monodeprotection, leading to the precursor with a very poor yield.

Accordingly, it is an object of the present invention to provide alternative to conventional cryptophanes. Inventors have found that compounds of formula (I) of the invention have enhanced solubility compared to conventional cryptophanes.

Another aim of the present invention is to provide compounds that are easily and selectively functionalized.

The presence of one or more nitrogen atoms on the cages of the invention enable an easy and effective functionalization, and also if need bimodal functionalization with more than one target compounds.

The enhanced solubility of the aza-cryptophanes has also enabled to prepare nanoemulsions thanks to said aza-cryptophanes, which is another aim of the present invention.

Thus, in one aspect, the present invention relates to a compound of following formula (I):

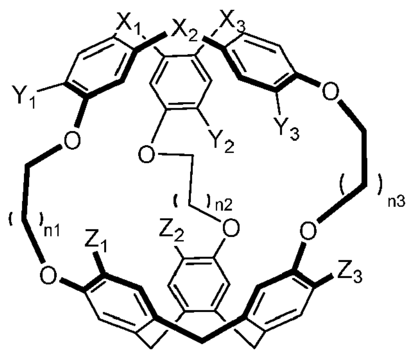

(I),

wherein:

$X_1$, $X_2$ and $X_3$ are independently selected from $-CH_2-$ and -NR-, at least one of $X_1$, $X_2$ and $X_3$ representing -NR-;
R is chosen from:

- H;
- a $C_1$-$C_6$ alkyl;
- a $C_2$-$C_6$ alkenyl;
- a $C_2$-$C_6$ alkynyl;
- a LZ group;
- an *ad hoc* protecting group, in particular a benzyl, *p*-methoxybenzyl, 2-tetrahydropyranyl (THP), a carbamate, for example Boc, Fmoc, Troc, Cbz, a silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS), or 2-(trimethylsilyl)ethoxymethyl (SEM), or an acyl group, in particular an acetyl;

$Y_1$, $Y_2$ and $Y_3$ are independently selected from:

- H,

- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- a O-L'Z' group;
- OH or a O-P' group, wherein P' is an *ad hoc* protecting group, in particular a benzyl; *p*-methoxybenzyl; silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS); 2-(trimethylsilyl)ethoxymethyl (SEM); allyl; methoxymethyl (MOM) ; tetrahydropyran (THP) or an acyl group, in particular an acetyl;

$Z_1$, $Z_2$ and $Z_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- a O-L"Z" group;
- OH or a O-P" group, wherein P" is an *ad hoc* protecting group, in particular a benzyl; *p*-methoxybenzyl; silyl, in particular a benzyl; *p*-methoxybenzyl; silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS); 2-(trimethylsilyl)ethoxymethyl (SEM); allyl; methoxymethyl (MOM) ; tetrahydropyran (THP) or an acyl group, in particular an acetyl;

n1, n2 and n3 are independently chosen from 0, 1, 2 and 3;
L, L' and L" are independently from each other a linker;
Z, Z' and Z" are independently from each other:

- a terminal reactive function or group, in particular able to form a covalent bond with a peptide, a protein, an antibody or a fragment thereof, a carrier, a solid support, a multivalent scaffold; an anchor; a dye or a fluorescent residue; or
- a peptide, a protein, an antibody or a fragment thereof, a carrier, a solid support, a multivalent scaffold; an anchor; a dye or a fluorescent residue;

or a pharmaceutically acceptable salt thereof.

In a particular embodiment, R is chosen from:

- H;
- a LZ group;
- an *ad hoc* protecting group, in particular a benzyl, *p*-methoxybenzyl, 2-tetrahydropyranyl (THP), a carbamate, for example Boc, Fmoc, Troc, Cbz, a silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS), or 2-(trimethylsilyl)ethoxymethyl (SEM), or an acyl group, in particular an acetyl.

In a particular embodiment, $Y_1$, $Y_2$ and $Y_3$ are independently selected from:

- H,
- a O-$C_1$-$C_6$ alkyl;
- a O-L'Z' group;
- OH or a O-P' group, wherein P' is an *ad hoc* protecting group, in particular a benzyl; *p*-methoxybenzyl; silyl, in particular a benzyl; p-methoxybenzyl; silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS); 2-(trimethylsilyl)ethoxymethyl (SEM); allyl; methoxymethyl (MOM) ; tetrahydropyran (THP) or an acyl group, in particular an acetyl.

In a particular embodiment, $Z_1$, $Z_2$ and $Z_3$ are independently selected from:

- H,
- a O-$C_1$-$C_6$ alkyl;
- a O-L"Z" group;
- OH or a O-P" group, wherein P" is an *ad hoc* protecting group, in particular a benzyl; p-methoxybenzyl; silyl, in particular a benzyl; *p*-methoxybenzyl; silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS); 2-(trimethylsilyl)ethoxymethyl (SEM); allyl; methoxymethyl (MOM) ; tetrahydropyran (THP) or an acyl group, in particular an acetyl.

In a particular embodiment, $Y_1$, $Y_2$ and $Y_3$ are H.
In a particular embodiment, $Z_1$, $Z_2$ and $Z_3$ are H, or a O-$C_1$-$C_6$ alkyl, in particular OMe.
In a particular embodiment, $X_1$ is -NR-, and $X_2$ and $X_3$ are -$CH_2$-.
In a particular embodiment, $X_1$ and $X_2$ are independently selected from -NR- groups, and $X_3$ is -$CH_2$-.
In a particular embodiment, $X_1$, $X_2$ and $X_3$ are independently selected from -NR-groups.
In a particular embodiment, $X_1$ is -NH- or -N(LZ)-, and $X_2$ and $X_3$ are -$CH_2$-.
In a particular embodiment, $X_1$ and $X_2$ are independently selected from -NH- and -N(LZ)- groups, with notably $X_1$ being -N(LZ)- and $X_2$ being -NH-, and $X_3$ is -$CH_2$-
**[0023]** In a particular embodiment, $X_1$, $X_2$ and $X_3$ are independently selected from -NH- and-N(LZ)- groups, with notably $X_1$ being -N(LZ)- and $X_2$ and $X_3$ being -NH-, or $X_1$ and $X_2$ chosen from -N(LZ)- groups, and $X_3$ being -NH-.
In a particular embodiment, n1, n2 and n3 are independently chosen from 0, 1 and 2.
In a particular embodiment, n1, n2 and n3 are such as n1 = n2 = n3.
In a more particular embodiment, n1 = n2 = n3 = 0.
In a more particular embodiment, n1 = n2 = n3 = 1.
In a more particular embodiment, n1 = n2 = n3 = 2.
In another particular embodiment, n1, n2 and n3 are not such as n1 = n2 = n3.
In a more particular embodiment, n1 = n2 ≠ n3; or n1 ≠ n2 = n3; or n1 = n3 ≠ n2; or n1 ≠ n2 ≠ n3.
In an even more particular embodiment, n1, n2 and n3 are such as:

- n1 = n2 = 0 and n3= 1; or
- n1=0 and n2 = n3 = 1; or
- n1 = n2 = 1 and n3= 2.

In a particular embodiment, $X_1$ is -NR- with R being LZ, $X_2$ and $X_3$ being in particular -$CH_2$- or -NH-.
In a particular embodiment, $Y_1$ is O-L'Z', $Y_2$ and $Y_3$ being in particular H.
In a particular embodiment, $Z_1$ is O-L"Z", $Z_2$ and $Z_3$ being in particular a O-$C_1$-$C_6$ alkyl, more particularly OMe.
In a particular embodiment, $X_1$, $X_2$, $X_3$, $Y_1$, $Y_2$, $Y_3$, $Z_1$, $Z_2$, $Z_3$, are such that the compound of formula (I) of the invention bears no LZ, L'Z' or L"Z" group.
In a particular embodiment, $X_1$, $X_2$, $X_3$, $Y_1$, $Y_2$, $Y_3$, $Z_1$, $Z_2$, $Z_3$, are such that the compound of formula (I) of the invention bears one LZ, L'Z' or L"Z" group.
In a particular embodiment, $X_1$, $X_2$, $X_3$, $Y_1$, $Y_2$, $Y_3$, $Z_1$, $Z_2$, $Z_3$, are such that the compound of formula (I) of the invention bears two LZ groups; or one LZ group and one L'Z' or L"Z" group.
In a particular embodiment, L is $(W)_i$-$L_1$-$(F_1$-$L_2)_j$-$(F_2)_k$- and/or L' is $(W')_i$-$L'_1$-$(F'_1$-$L'_2)_j$-$(F'_2)_k$-, wherein:

- W and W' are independently chosen from -C(=O)-, -C(=O)O-, -C(=O)NH-,-C(=S)-O-, notably -C(=O)- ;
- $L_1$, $L_2$, $L'_1$ and $L'_2$ are independently is a $C_1$-$C_{12}$ alkane diyl, a $C_2$-$C_{12}$ alkene diyl or a $C_2$-$C_{12}$ alkyne diyl chain optionally interrupted by one or more heteroatoms, notably selected from an oxygen atom, a sulphur atom or a nitrogen atom, said nitrogen and sulphur atoms being optionally oxidized, for example -$(CH_2)_m$O-$(CH_2$-$CH_2$-$O)_{m'}$-, wherein m and m' are independently from 1 to 6;
- $F_1$, $F_2$, $F'_1$ and $F'_2$ are independently chosen from -C(=O)-, -C(=O)-C(=O)-, -C(=O)-C(=O)-NH-, -NHC(=O)-C(=O)-, -NHC(=O)-C(=O)-NH-, -C(=O)-C(H)=N-NH-, -NH-C(=O)-C(H)=N-NH-, ester, amide, amine, -$CH_2$-, ether, thioether, imine, succinimide, thio-succinimide, oxime, hydrazone, hydrazonamide, -C(=O)CH2-NH-, -NH-CH2-C(=O)-, triazole functions or groups;
- i, j, and k are independently 0 or 1.

In a particular embodiment, k is 0, Z being in particular a terminal reactive function or group, in particular able to form a covalent bond with a peptide, a protein, a carrier, a solid support, a multivalent scaffold; an anchor; a dye or a fluorescent residue.
In a particular embodiment, k is 1, Z being in particular a peptide, a protein, a carrier, a solid support, a multivalent scaffold; an anchor; a dye or a fluorescent residue.

In a particular embodiment, Z is a terminal reactive function or group, more particularly a halogen, in particular Cl, Br, I, F, more particularly Br, $C_2$-$C_6$ alkenyl, $C_2$-$C_6$ alkynyl, azido, epoxide, C(=O)H, hemiacetal, $C(=O)R_c$, acetal, $SR_a$, $NH_2$ or $NHC(=O)CH_2Hal$, wherein Hal is a halogen, in particular Cl, Br, I, F, more particularly Br, N-maleimide.

In a particular embodiment, LZ and/or L'Z' and/or L"Z" is chosen from $-CH_2-C\equiv CH$, $-(CH_2)_k$-triazole, wherein k is from 1 to 6, and $-C(=O)-(CH_2)_n-Hal$, wherein n is from 1 to 6, in particular 3 and Hal is halogen, in particular Cl, Br, I, F, more particularly Br.

In a particular embodiment, the compound of the invention is chosen from the following formulae:

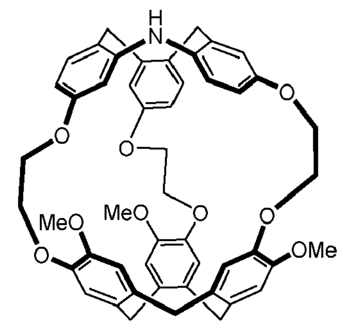
,
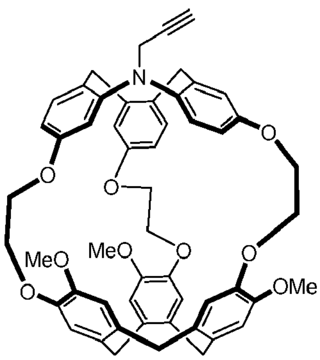
,
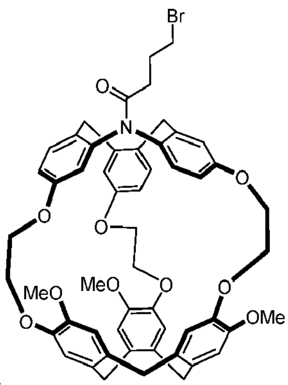
,

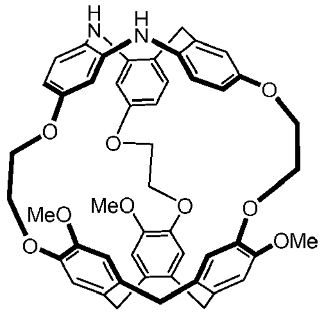
,
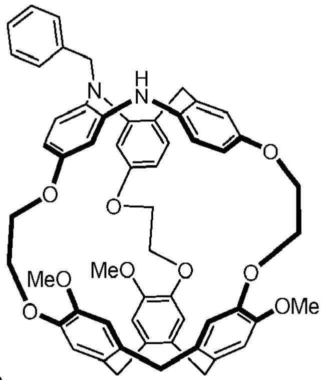
,
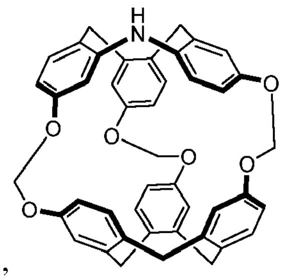
,

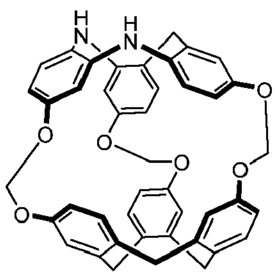
,
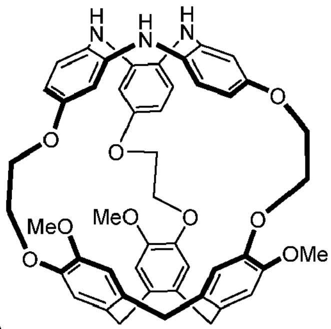
,
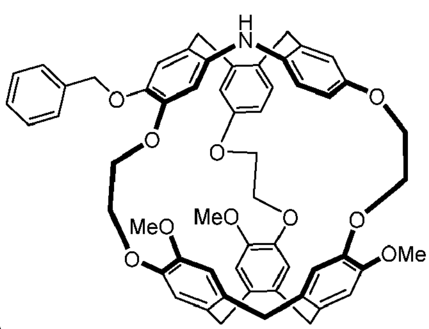
.

The present invention also relates to a process of preparation of a compound of formula (I) as defined above, wherein n1, n2 and n3 are independently chosen from 1, 2 and 3, in particular from 1 and 2, comprising a step (i) of contacting a compound of following formula (II) with formic acid, $P_2O_5$, $HClO_4$, or $Sc(OTf)_3$:

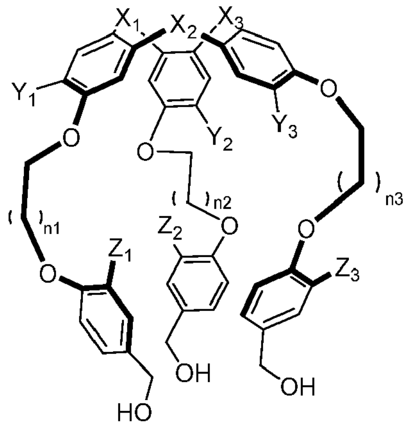

(II),

wherein :

$X_1$, $X_2$ and $X_3$ are independently selected from $-CH_2-$ and -NR-, at least one of $X_1$, $X_2$ and $X_3$ representing -NR-; R is chosen from:

- H;
- a $C_1$-$C_6$ alkyl;
- a $C_2$-$C_6$ alkenyl;
- a $C_2$-$C_6$ alkynyl;
- an *ad hoc* protecting group, in particular a benzyl, *p*-methoxybenzyl, 2-(trimethylsilyl)ethoxymethyl (SEM), 2-tetrahydropyranyl (THP), a carbamate, for example Boc, Fmoc, Troc, Cbz, a silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS), or 2-(trimethylsilyl)ethoxymethyl (SEM), or an acyl group, in particular an acetyl;

$Y_1$, $Y_2$ and $Y_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- optionally a O-L'Z' group;
- OH or a O-P" group, wherein P" is an *ad hoc* protecting group, in particular a benzyl; *p*-methoxybenzyl; silyl, in particular a benzyl; p-methoxybenzyl; silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS); 2-(trimethylsilyl)ethoxymethyl (SEM); allyl; methoxymethyl (MOM) ; tetrahydropyran (THP) or an acyl group, in particular an acetyl;

$Z_1$, $Z_2$ and $Z_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- optionally a O-L"Z" group;
- OH or a O-P" group, wherein P" is an *ad hoc* protecting group, in particular a benzyl; *p*-methoxybenzyl; silyl, in particular a benzyl; *p*-methoxybenzyl; silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS); 2-(trimethylsilyl)ethoxymethyl (SEM); allyl; methoxymethyl (MOM) ; tetrahydropyran (THP) or an acyl group, in particular an acetyl;

L' and L" are independently from each other a linker;

Z' and Z" are independently from each other:

- a terminal reactive function or group, in particular able to form a covalent bond with a peptide, a protein, an antibody or a fragment thereof, a carrier, a solid support, a multivalent scaffold; an anchor; a dye or a fluorescent residue; or
- a peptide, a protein, an antibody or a fragment thereof, a carrier, a solid support, a multivalent scaffold; an anchor; a dye or a fluorescent residue;

n1, n2 and n3 are independently chosen from 0, 1, 2 and 3.

The -OH groups of compound of formula (II) can be protected by an *ad hoc* protecting group known from the skilled in the art, for example a THP group, and deprotected prior to the reaction yielding compound (II) as defined above.

In a particular embodiment, the compound of formula (II) is obtained by contacting a compound of following formula (III) with a compound of following formula (IV), in presence of a base, in particular a carbonate, more particularly $Cs_2CO_3$:

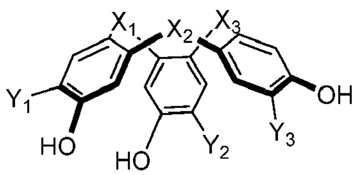 (III) ; 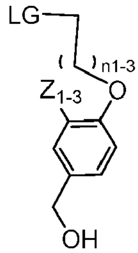 (IV),

wherein LG is a leaving group, in particular chosen from halogens, more particularly I, Br, Cl, notably I, and O-tosyl.

The -OH group of compound (IV) can be protected by an *ad hoc* protecting group known from the skilled in the art, for example a THP group.

When $Z_1$=$Z_2$=$Z_3$ and n1=n2=n3, the compound of formula (IV) corresponds to a single compound of following formula:

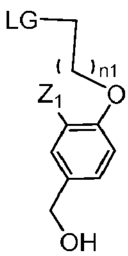 .

When this is not the case, the compound of formula (IV) is a mixture of compounds as follows:

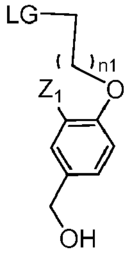 , 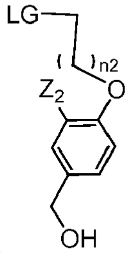 , 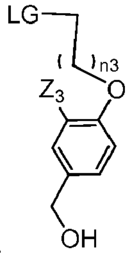 ,

said compounds being in particular introduced as a mixture or sequentially for the reaction with the compound of formula (III).

In a particular embodiment, the compound of formula (III) is obtained by contacting a compound of following formula (V) with BBr3:

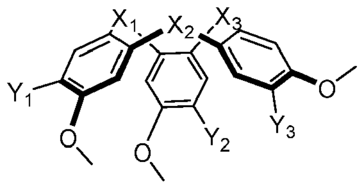

(V).

In a particular embodiment, the compound of formula (V), when $X_1$ is $-CH_2-$ or -NR-, $X_2$ is -NR- and $X_3$ is $-CH_2-$, is obtained by contacting a compound of following formula (VI) with $HClO_4$:

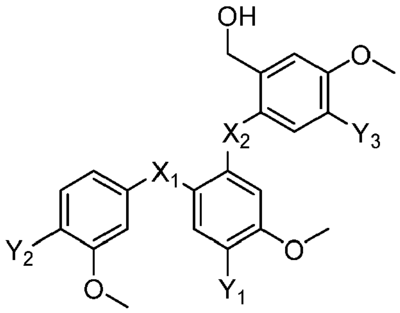

(VI),

wherein $X_1$ is $-CH_2-$ or -NR-, and $X_2$ is -NR-.

In a particular embodiment, the compound of formula (V), when $X_1$ and $X_2$ are independently chosen from -NR- groups, R being not H, is obtained by from a compound of following formula (VII), in particular by Buchwald amination:

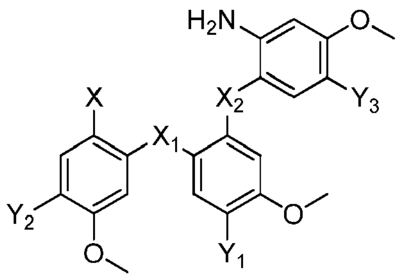

(VII),

wherein $X_1$ and $X_2$ are independently chosen from -NR- groups, R being not H, in particular - NP- groups, with P as defined above, and X is an halogen atom, in particular Br, I or Cl.

In a particular embodiment, the invention concerns a process of the invention of preparation of a compound of formula (I) wherein $X_1$, $X_2$ and/or $X_3$, in particular $X_1$ and/or $X_2$, is -N(-LZ)-, said process comprising after step (i) a step of contacting a compound of formula (I) wherein $X_1$, $X_2$ and/or $X_3$, in particular $X_1$ and/or $X_2$, is -NH- with a compound of formula LG-LZ, wherein LG is a leaving group, LG being in particular a halogen, more particularly Cl, Br, I, F, preferably Br, or a O-tosyl group.

In a particular embodiment, the invention concerns a process of the invention of preparation of a compound of formula (I) wherein $Y_1$, $Y_2$ and/or $Y_3$, in particular $Y_1$ and/or $Y_2$, is -O-L'Z', said process comprising after step (i) a deprotection step of a compound of formula (I) with $Y_1$, $Y_2$ and/or $Y_3$, in particular $Y_1$ and/or $Y_2$, being -OP', followed by the contacting the obtained compound of formula (I) wherein $Y_1$, $Y_2$ and/or $Y_3$, in particular $Y_1$ and/or $Y_2$, is -OH with a compound of formula LG'-L'Z', wherein LG' is a leaving group, LG' being in particular a halogen, more particularly Cl, Br, I, F, preferably Br, or a O-tosyl group.

In a particular embodiment, the invention concerns a process of the invention of preparation of a compound of formula (I) wherein $Z_1$, $Z_2$ and/or $Z_3$, in particular $Z_1$ and/or $Z_2$, is -O-L"Z", said process comprising after step (i):

- a deprotection step of a compound of formula (I) with $Z_1$, $Z_2$ and/or $Z_3$, in particular $Z_1$ and/or $Z_2$, being -OP", or
- a dealkylation step of a compound of formula (I) with $Z_1$, $Z_2$ and/or $Z_3$, in particular $Z_1$ and/or $Z_2$, being $-O-C_1-C_6$ alkyl, notably OMe, in particular in presence of iodotrimethylsilane, hydrobromic acid, boron tribromide or aluminium chloride.

followed by the contacting the obtained compound of formula (I) wherein $Z_1$, $Z_2$ and/or $Z_3$, in particular $Z_1$ and/or $Z_2$, is -OH with a compound of formula LG"-L"Z", wherein LG" is a leaving group, LG" being in particular a halogen, more particularly Cl, Br, I, F, preferably Br, or a O-tosyl group.

In a particular embodiment, the invention concerns a process of the invention of preparation of a compound of formula (I) as defined above, wherein n1, n2 and n3 are 0, comprising a step (i') of contacting a compound of formula (III) with a compound of following formula (VII), in presence of $BrCH_2Cl$ and a base, in particular $Cs_2CO_3$:

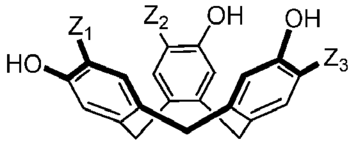

(VII),

wherein $Z_1$, $Z_2$ and $Z_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- optionally a O-L'Z' group;
- a O-P" group, wherein P" is an *ad hoc* protecting group, in particular a benzyl; *p*-methoxybenzyl; silyl, in particular a benzyl; *p*-methoxybenzyl; silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS); 2-(trimethylsilyl)ethoxymethyl (SEM); allyl; methoxymethyl (MOM) ; tetrahydropyran (THP) or an acyl group, in particular an acetyl.

In a particular embodiment, the invention concerns a process of the invention of preparation of a compound of formula (I) as defined above, wherein n1, n2 and n3 are 0, comprising a step (i') of contacting a compound of formula (III), with $ClCH_2SMe$, then $SOCl_2$, and then with a compound of following formula (VII), in presence of a base, in particular $Cs_2CO_3$.

In another aspect, the invention concerns a compound of one of the following formulae:

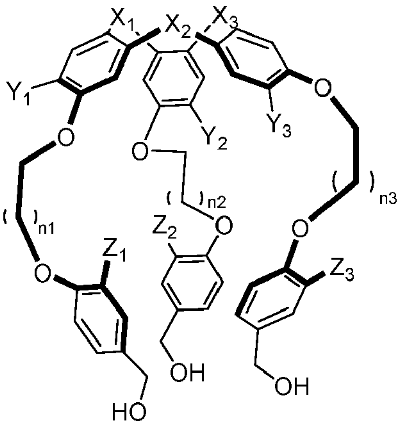

(II),

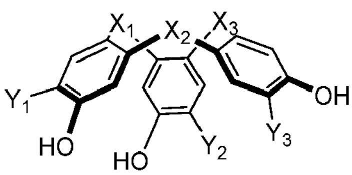

(III),

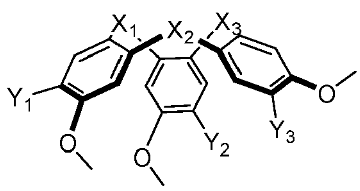

(V).

In another aspect, the invention concerns a complex constituted of or comprising a compound of formula (I) complexed with a hyperpolarized noble element, in particular Xe, more particularly $^{129}Xe$.

By "hyperpolarized" is in particular meant a certain noble gas, the polarization of the nuclei of which being artificially enhanced over the natural levels at thermal equilibrium. Techniques that enable this hyperpolarization are well known for the skilled in the art.

In a particular embodiment, the invention concerns a compound of formula (I) as defined above or a complex as defined above for its use as an in vivo diagnostic tool, in particular in NMR spectroscopy or in MRI applications.

In another aspect, the invention concerns an oil-in-water nanoemulsion comprising:

- An oil;
- An aqueous phase;
- Optionally, a surfactant;
- Optionally, a co-surfactant and/or a solubilizier; and
- A compound of following formula (Ia):

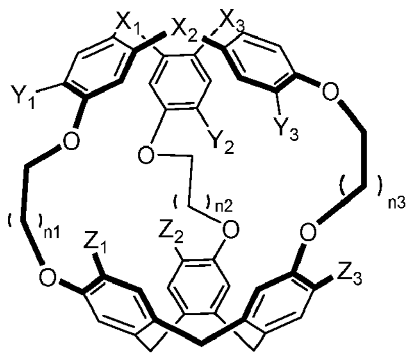

(Ia)

Wherein:

$X_1$, $X_2$ and $X_3$ are independently selected from $-CH_2-$ and -NR-, at least one of $X_1$, $X_2$ and $X_3$ representing -NR-; R is chosen from:

- H;
- a $C_1$-$C_6$ alkyl;
- a $C_2$-$C_6$ alkenyl;
- a $C_2$-$C_6$ alkynyl;
- an *ad hoc* protecting group, in particular a benzyl, *p*-methoxybenzyl, 2-tetrahydropyranyl (THP), a carbamate, for example Boc, Fmoc, Troc, Cbz, a silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS), or 2-(trimethylsilyl)ethoxymethyl (SEM), or an acyl group, in particular an acetyl;

$Y_1$, $Y_2$ and $Y_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- OH;

$Z_1$, $Z_2$ and $Z_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;

- OH;

n1, n2 and n3 are independently chosen from 0, 1, 2 and 3,said compound of formula (I) being optionally complexed with a hyperpolarized noble element, in particular Xe, more particularly $^{129}Xe$.

The oil-in-water nanoemulsions of the invention may in particular be formed by spontaneous emulsification.
The term "nanoemulsion" refers in particular to oil-in-water dispersions comprising nanometer scale oil phase droplets in an aqueous phase.
The diameter of the nanoemulsion oil phase droplets is in particular from 20 to 300 nm, preferably from 25 to 100 nm.
Size distributions can be determined for example by the method of dynamic light scattering.
The oil-in-water nanoemulsions of the invention are preferably made from excipients, which are Generally Recognized As Safe (GRAS) ingredients.
In a particular embodiment, the oil is a natural oil, more particularly a vegetable oil or an animal oil; a synthetic oil; or a mixture thereof. For example, the synthetic oil can be medium chain triglycerides such as Labrafac® WL1349.
In a particular embodiment, the aqueous phase consists of or comprises water or an aqueous buffer. Such an aqueous buffer may be useful to control the pH and the osmolarity of the preparation.
The surfactant may be selected as well known by the skilled in the art. In a particular embodiment, the surfactant is chosen from anionic surfactants, nonionic surfactants, cationic surfactants, amphoteric surfactants and (extended) surfactants containing a non-ionic spacer-arm central extension and an ionic or nonionic polar group. For example, the surfactant can be a PEGlygated non-ionic surfactant such as hydroxystearate PEG15 (Kolliphor® HS15).
The co-surfactant may be selected as defined above for the surfactant.
The solubilizier may be selected as well known by the skilled in the art. It can be selected from glycerides, or can be a solvent such as dimethyl sulfoxide, ethanol or a derivate, and in particular diethylene glycol monoethyl ether (Transcutol® HP).
The co-surfactant and/or a solubilizier may be useful to optimize if needed the stability and the drug-loading of the nanodroplets.

**Definitions**

The following terms and expressions contained herein are defined as follows:
As used herein, a range of values in the form "x-y" or "x to y", or "x through y", include integers x, y, and the integers therebetween. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.
As used herein, the term "alkyl" refers to a straight-chain, or branched alkyl group having 1 to 6 carbon atoms, such as methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, hexyl, etc. The alkyl moiety of alkyl-containing groups, such as aralkyl or O-alkyl groups, has the same meaning as alkyl defined above. Lower alkyl groups, which are preferred, are alkyl groups as defined above which contain 1 to 4 carbons. A designation such as "C1-C4 alkyl" refers to an alkyl radical containing from 1 to 4 carbon atoms.
As used herein, the term "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.
All other terms used in the description of the present invention have their meanings as is well known in the art.
In another aspect, the present invention is directed to pharmaceutically acceptable salts of the compounds described above. As used herein, "pharmaceutically acceptable salts" includes salts of compounds of the present invention derived from the combination of such compounds with non-toxic acid.
Acid addition salts include inorganic acids such as hydrochloric, hydrobromic, hydroiodic, sulfuric, nitric and phosphoric acid, as well as organic acids such as acetic, citric, propionic, tartaric, glutamic, salicylic, oxalic, methanesulfonic, para-toluenesulfonic, succinic, and benzoic acid, and related inorganic and organic acids.
In addition to pharmaceutically-acceptable salts, other salts are included in the invention. They may serve as intermediates in the purification of the compounds, in the preparation of other salts, or in the identification and characterization of the compounds or intermediates.
The pharmaceutically acceptable salts of compounds of the present invention can also exist as various solvates, such as with water, methanol, ethanol, dimethylformamide, ethyl acetate and the like. Mixtures of such solvates can also be prepared. The source of such solvate can be from the solvent of crystallization, inherent in the solvent of

preparation or crystallization, or adventitious to such solvent. Such solvates are within the scope of the present invention.

It is recognized that compounds of the present invention may exist in various stereoisomeric forms. As such, the compounds of the present invention include both diastereomers and enantiomers. The compounds are normally prepared as racemates and can conveniently be used as such, but individual enantiomers can be isolated or synthesized by conventional techniques if so desired. Such racemates and individual enantiomers and mixtures thereof form part of the present invention.

It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; Wilen, S.H. Stereochemistry of Organic Compounds; Wiley: New York, 1994, and Jacques, J, et al. Enantiomers, Racemates, and Resolutions; Wiley: New York, 1981, each incorporated by reference herein in their entireties.

Synthesis

The compounds of the present invention may be prepared in a number of methods well known to those skilled in the art, including, but not limited to those described below, or through modifications of these methods by applying standard techniques known to those skilled in the art of organic synthesis. The appropriate modifications and substitutions will be readily apparent and well known or readily obtainable from the scientific literature to those skilled in the art. In particular, such methods can be found in R.C. Larock, Comprehensive Organic Transformations, Wiley-VCH Publishers, 1999.

All processes disclosed in association with the present invention are contemplated to be practiced on any scale, including milligram, gram, multigram, kilogram, multikilogram or commercial industrial scale.

It will be appreciated that the compounds of the present invention may contain one or more asymmetrically substituted carbon atoms, and may be isolated in optically active or racemic forms. Thus, all chiral, diastereomeric, racemic forms, isomeric forms of a structure are intended, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described in the present specification, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, 3rd ed., John Wiley and Sons, 1999; J. F. W. McOmie in Protective Groups in Organic Chemistry, Plenum Press, 1973.

**DRAWINGS**

**Figure 1** illustrates the $^{129}Xe$ NMR spectrum (5 bars, $CDCl_3$) of cryptophane-IN **12.**
**Figure 2** illustrates the 3D structure of cryptophane-IN **12** determined by RX crystallography.

**EXAMPLES**

**General experimental procedure**

All reagents and solvents were obtained from commercial suppliers and were used without further purification. Purification was carried out according to standard laboratory methods. Solvents for purification purposes were used as obtained from suppliers without further purification. NMR spectra were recorded at 400 MHz (Bruker Advance III 400 MHz) for 1 H NMR and at 100 or 125MHz for 13C NMR in chloroform-d with chemical shift (δ) given in parts per million (ppm) relative to TMS as internal standard and recorded at 295K. The following abbreviations are used to describe peak splitting patterns when appropriate: br=broad, s=singlet, d=doublet, t=triplet, q=quartet, m=multiplet, dd=doublet of doublet, dt=doublet of triplet. Coupling constants J are reported in hertz units (Hz). Chromatography was carried out on a column using silica gel 60 Merck (0.063-0.200 mm) as the stationary phase. Melting points were measured on a Stuart Automatic Melting Point SMP-50 apparatus. High resolution mass spectra (HRMS) were obtained by electronic impact (HRMS/EI), or by electrospray (HRMS/ ESI) on a Bruker maXis mass spectrometer.

**General procedures**

Synthesis of tosylhydrazones - General procedure A

In a round bottom flask, para-toluene sulfonylhydrazide (1.05 equiv.) was solubilized in methanol (4 mL/g). Substituted benzaldehyde (1.00 equiv.) was added to the solution, the resulting mixture was stirred vigorously for 2h30. The resulting mixture was evaporated to dryness, dissolved in a small portion of diethyl ether. The solid was filtered, and the expected product was obtained as a white powder.

Synthesis for the Barluenga Boronic Coupling - General procedure B

In a round bottom flask, substituted para-toluene sulfonylhydrazone (1.00 equiv.) was solubilized in dioxane (15 mL/mmol). Potassium carbonate (4 equiv.) and boronic acid (1.5 equiv.) were added to the solution, and the reaction mixture was heated at reflux for 2h30. The resulting mixture was allowed to reach room temperature, an aqueous saturated solution of sodium bicarbonate was added (15 mL/ mmol), and the mixture was extracted three times with ethyl acetate. The organic layers were combined, dried over $MgSO_4$, filtered and evaporated under vacuum. The crude mixture was purified using silica chromatography and a mixture of cyclohexane and ethyl acetate as eluent (95/5), affording the expected product.

**Example 1: Synthesis of 1N-cryptophane of the invention**

**N'-[(1*E*)-(2-bromo-4-methoxyphenyl)methylidene]-4-methylbenzene-1-sulfonohydrazide 1**

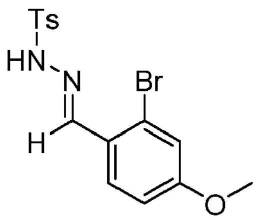

Prepared from 2-bromo-4-methoxybenzaldehyde (10.00 g, 46.53 mmol), following general procedure A, the expected compound was obtained as a white powder (17.39 g, 97%).
$^1H$ NMR (400 MHz, $CDCl_3$): δ = 8.06 (s, 1H), 7.90 - 7.79 (m, 4H), 7.32 (d, J = 8.0 Hz, 2H), 7.03 (d, J = 2.5 Hz, 1H), 6.85 (m, 1H), 3.81 (s, 3H), 2.41 (s, 3H).
$^{13}C$ NMR (100 MHz, $CDCl_3$): δ = 161.7, 146.4, 144.5, 135.4, 129.9, 128.8, 128.1, 125.0, 124.8, 117.7, 114.6, 55.8, 21.8.
Melting point: 149°C

**1 -Bromo-3-methoxy-6-(3-methoxybenzyl)-benzene 2**

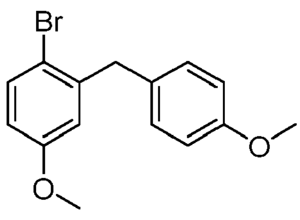

Prepared from tosylhydrazone **1** (17.4 g, 45.4 mmol) and 3-methoxyphenylboronic acid (8.97 g, 59 mmol),

following general procedure B, the expected compound was obtained as a light yellow oil (11.7 g, 84 %).
**$^1$H NMR** (400 MHz, $CDCl_3$): δ = 7.21 (t, J = 7.8 Hz, 1H), 7.13 (d, J = 2.6 Hz, 1H), 7.05 (d, J = 8.5 Hz, 1H), 6.81-6.72 (m, 4H), 4.03 (s, 2H), 3.78 (s, 3H), 3.77 (s, 3H).
**$^{13}$C NMR** (100 MHz, $CDCl_3$): δ = 159.8,158.7,141.8,132.3,131.5,129.5,125.0,121.4,118.0, 114.8, 113.8, 111.5, 55.7, 55.3, 41.0.
**HRMS-ESI** calculated for $C_{15}H_{15}BrO_4$ [$M^{+\cdot}$], 306.0255 found 306.0253

**(2-Amino-5-methoxyphenyl)methanol 3**

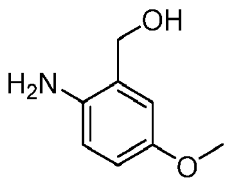

Obtained according to the procedure described by Wales et al. (Org. Lett. 2019, 21 (12), 4703-4708).

**2-[(Tert-butyldimethylsilyl)oxymethyl]-4-methoxyaniline 4**

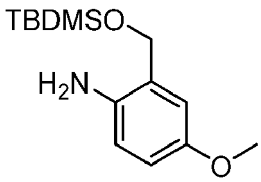

**3** (1.3 g, 8.55 mmol), TBDMSC1 (1.6 g, 12.8 mmol) and imidazole (872 mg, 12.8 mmol) were dissolved in dichloromethane (20 mL) and the solution was stirred at room temperature. After completion, the reaction was quenched water, extracted with ethyl acetate, washed with brine, dried over $MgSO_4$ and concentrated under vacuum. Purification over silica gel (cyclohexane/$Et_2O$ 9/1 to 8/2) afforded the product as a red oil (1.37 g, 60%).
$^1$H NMR (400 MHz, $CDCl_3$): δ = 6.70 - 6.66 (m, 2H), 6.65 - 6.59 (m, 1H), 4.65 (s, 2H), 3.86 (s, 2H), 3.74 (s, 3H), 0.91 (s, 9H), 0.08 (s, 6H).
$^{13}$C NMR (100 MHz, $CDCl_3$): δ = 152.4, 139.5, 126.9, 116.9, 114.5, 113.8, 64.7, 55.9, 26.0 (3C),18.4, -5.1 (2C).
HRMS-ESI calculated for $C_{14}H_{25}NO_2Si$ [$M^{+\cdot}$] 267.1655, found 267.1657

**2-(((Tert-butyldimethylsilyl)oxy)methyl)-4-methoxy-N-(5-methoxy-2-(3-methoxybenzyl)phenyl)aniline 5**

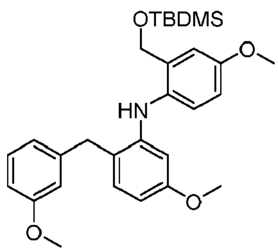

**2** (1 g, 3.26 mmol), **4** (872 mg, 3.26 mmol), $Pd(OAc)_2$ (74 mg, 0.326 mmol), Binap (406 mg, 0.652 mmol), cesium carbonate (3.7 g, 11.4 mmol) were added in toluene (30 mL) under nitrogen atmosphere and heated to reflux for 3h. The mixture was filtrated through a pad of celite using ethyl acetate as the eluent. The solvent was removed under vacuum and purification over silica gel (Cyclohexane/$Et_2O$ 95/5) afforded the product as a yellow oil. (1.4 g, 87%).
$^1$H NMR (400 MHz, $CDCl_3$): δ = 7.21 (t, J = 8.0 Hz, 1H), 7.05 (d, J = 8.7 Hz, 1H), 6.98 (d, J = 8.2 Hz, 1H), 6.93 (d, J =

3.0 Hz, 1H), 6.81 - 6.73 (m, 4H), 6.38 (dt, J = 8.2, 2.6 Hz, 2H), 5.67 (s, 1H), 4.44 (s, 2H), 3.89 (s, 2H), 3.79 (s, 3H), 3.76 (s, 3H), 3.69 (s, 3H), 0.89 - 0.86 (m, 9H), 0.03 - -0.01 (m, 6H).
$^{13}C$ NMR (100 MHz, $CDCl_3$): δ= 159.9, 159.5, 155.6, 144.8, 141.7, 134.9, 133.7, 131.5, 129.7, 123.4, 121.1, 120.0, 114.5, 113.6, 113.3, 111.8, 104.4, 101.5, 62.9, 55.7, 55.3, 55.2, 37.4, 26.0 (3C), 18.4, -5.1 (2C).
HRMS-ESI calculated for $C_{29}H_{39}NO_4$ [$M^{+\cdot}$] 493.2648, found 493.2647

**(5-methoxy-2-((5-methoxy-2-(3-methoxybenzyl)phenyl)amino)phenyl)methanol 6**

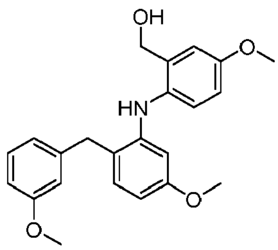

TBAF (1M in THF, 5.7 mL, 5.7 mmol) was added at 0°C to a solution of **5** (1.4 g, 2.84 mmol) in THF (25 mL) and the mixture was stirred at room temperature until completion of the reaction. The solution was then quenched with saturated $NaHCO_3$, extracted with ethyl acetate, washed with brine, dried over $MgSO_4$ and concentrated under vacuum. Purification over silica gel (Cyclohexane/$Et_2O$ 7/3 to 4/6) afforded the product as a light yellow oil. (1.034 g, 96%).
$^1H$ NMR (400 MHz, $CDCl_3$): δ = 7.25 - 7.19 (m, 1H), 7.12 (d, J = 8.6 Hz, 1H), 7.08 (d, J = 8.1 Hz, 1H), 6.79 (ddd, J = 8.6, 7.6, 2.6 Hz, 5H), 6.41 (dt, J = 8.1, 2.5 Hz, 2H), 5.82 (s, 1H), 4.32 (s, 2H), 3.94 (s, 2H), 3.77 (s, 3H), 3.75 (s, 3H), 3.71 (s, 3H).
$^{13}C$ NMR (100 MHz, $CDCl_3$): δ= 159.9, 159.5, 155.6, 144.9, 141.8, 134.3, 134.1, 131.9, 129.6, 124.0, 120.9, 119.4, 114.8, 114.4, 114.0, 111.7, 104.0, 101.2, 63.4, 55.6, 55.3, 55.2, 37.7. HRMS-ESI calculated for $C_{23}H_{26}NO_4$ [$M+H^+$] 380.1862, found 380.1843

**2,7,12-trimethoxy-10,15-dihydro-*5H*-tribenzo[*b*,*e*,*h*]azonine 7**

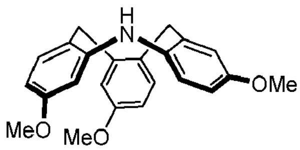

Perchloric acid (60%, 0.160 mL) was added dropwise to a solution of 6 (100 mg, 0.264 mmol) in acetonitrile (53mL) at 0°C and then the solution was heated to 60°C for 1h. The reaction was quenched with water, extracted with ethyl acetate, washed with brine, dried over $MgSO_4$ and concentrated under vacuum. Purification over silica gel (cyclohexane/$Et_2O$ 8/2 to 7/3) afforded the product as a white powder (85 mg, 89%).
$^1H$ NMR (400 MHz, $CDCl_3$): δ = 7.06 (d, *J* = 8.1 Hz, 1H), 6.87 - 6.81 (m, 2H), 6.72 - 6.64 (m, 3H), 6.50 (s, 1H), 6.34 (d, *J* = 8.4 Hz, 1H), 6.30 (s, 1H), 5.71 (s, 1H), 3.83 (s, 2H), 3.79 (s, 3H), 3.76 (s, 3H), 3.69 (s, 5H).
$^{13}C$ NMR (100 MHz, $CDCl_3$): δ = 159.0, 158.4 (2C), 155.4, 144.9, 141.4, 136.2, 134.0, 131.8, 130.9 (2C), 122.1, 116.9, 116.6, 113.1, 110.62, 106.29, 103.29, 55.51, 55.32, 55.30, 35.61, 35.47
HRMS-ESI calculated for $C_{23}H_{24}NO_3$ [$M+H^+$] 362.1756, found 362.1755
Melting point: 166-168 °C

**10,15-dihydro-5H-tribenzo[b,e,h]azonine-2,7,12-triol 8**

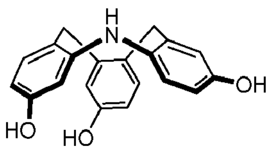

BBr3 (1M in DCM, 12.5 mL, 12.5 mmol) was added to a solution of 7 (450 mg, 1.25 mmol) in dichloromethane at 0°C and the mixture was stirred at room temperature until completion of reaction. The reaction was quenched at 0°C with water, extracted with ethyl acetate, washed with brine, dried over $MgSO_4$ and concentrated under vacuum. Purification over silica gel ($Et_2O$/cyclohexane 7/3) afforded the product as a light pink powder (383 mg, 96%).
$^1$H NMR (400 MHz, Acetone D-6): δ = 8.08 (s, 1H), 8.00 (s, 1H), 7.80 (s, 1H), 7.00 (d, *J* = 8.2 Hz, 2H), 6.83 (d, *J* = 8.5 Hz, 1H), 6.71 (d, *J* = 8.0 Hz, 2H), 6.62 (dd, *J* = 8.4, 2.7 Hz, 2H), 6.47 - 6.38 (m, 2H), 6.27 - 6.20 (m, 1H), 3.81 (s, 2H), 3.64 (s, 2H).
$^{13}$C NMR (100 MHz, $CDCl_3$): δ= 155.9, 155.4, 152.0, 145.5, 141.3, 135.4, 133.2, 131.4, 130.5, 130.3, 121.9, 117.4, 116.8, 115.7, 113.8, 111.9, 106.6, 103.7, 34.5, 34.5.
Melting point: 210-212 °C

**[4-(2-Bromoethoxy)-3-methoxyphenyl]methanol 9**

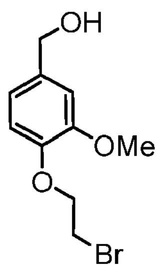

Obtained according to the procedure described by Spence et al. (J. Am. Chem. Soc. 2004, 126 (46), 15287-15294*)*.

**[4-(2-Iodoethoxy)-3-methoxyphenyl]methanol 10**

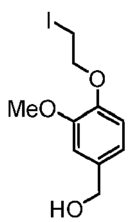

Obtained according to the procedure described by Spence et al. (J. Am. Chem. Soc. 2004, 126 (46), 15287-15294*)*.

**(((((10,15-Dihydro-5H-tribenzo[b,e,h]azonine-2,7,12-triyl)tris(oxy))tris(ethane-2,1-diyl))tris(oxy))tris(3-methoxybenzene-4,1-diyl))trimethanol 11**

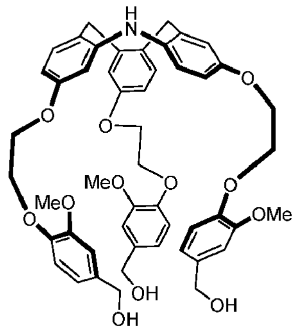

**10** (866 mg, 2.82 mmol) and cesium carbonate (916 mg, 2.82 mmol) was added to a solution of **8** (150 mg, 0.47 mmol) in dry DMF (10 mL) placed under nitrogen atmosphere. The mixture was stirred overnight at 80°C, and allowed to reach room temperature. The reaction was quenched with water and extracted with ethyl acetate. The organic layers were combined, washed with brine, dried over $MgSO_4$ and concentrated under vacuum. Purification over silica gel (AcOEt/MeOH, 100/0 to 95/5) afforded the product as a white solid (275 mg, 68%).

$^1$H NMR (400 MHz, $CDCl_3$): δ = 6.98 (dd, *J* = 16.8, 6.2 Hz, 1H), 6.93 - 6.75 (m, 11H), 6.73 (d, *J* = 2.5 Hz, 1H), 6.70 - 6.65 (m, 2H), 6.51 (s, 1H), 6.34 (s, 1H), 6.32 (s, 1H), 4.63 - 4.55 (m, 6H), 4.37 - 4.28 (m, 6H), 4.25 (t, *J* = 5.1 Hz, 4H), 4.18 (t, *J* = 4.8 Hz, 2H), 3.85 - 3.81 (m, 6H), 3.80 (s, 3H),3.78 (s, 2H), 3.64 (s, *J* = 12.0 Hz, 2H).

$^{13}$C NMR (100 MHz, $CDCl_3$): δ = 157.97, 157.42, 154.26, 149.85, 149.68, 147.64, 147.59, 147.52, 144.84, 141.38, 136.50, 134.83, 134.7, 134.68, 133.90, 132.15, 131.47, 130.90, 121.97, 119.65, 119.46, 119.41, 118.27, 117.88, 117.30, 114.29, 114.12, 114.09, 113.80, 111.52, 111.15, 111.13, 111.05, 110.83, 106.90, 104.32, 71.47, 67.92, 66.66, 66.46, 66.42, 65.23, 65.21, 65.18, 61.26, 55.99, 55.93, 55.83, 35.52, 35.41.

HRMS-ESI calculated for $C_{50}H_{53}NO_{12}Na$ [M+Na$^+$] 882.3465, found 882.3474

Melting point: 140-142 °C

**Cryptophane-1N 12**

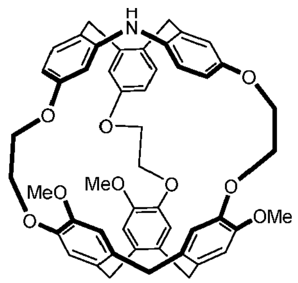

Formic acid (300 mL) was added to a solution of **11** (270 mg, 0.313 mmol) in chloroform (15 mL) and the reaction mixture was heated for 3h at 55°C. The solvent was removed under vacuum and purification over silica gel (DCM/$Et_2O$ 95/5 to 80/20) to afford the product as a light pink powder (182 mg, 72%).

$^1$H NMR (400 MHz, $CDCl_3$): δ = 7.13 (d, *J* = 8.6 Hz, 1H), 7.05 (dd, *J* = 8.4, 6.1 Hz, 2H), 6.80 (dd, *J* = 4.6, 2.7 Hz, 2H), 6.72 (d, *J* = 2.6 Hz, 1H), 6.69 (s, 1H), 6.67 - 6.63 (m, 4H), 6.59 (s, 1H), 6.38 (dd, *J* = 8.7, 2.9 Hz, 1H), 6.33 (dt, *J* = 8.4, 2.4 Hz, 2H), 4.64 - 4.56 (m, 5H), 4.42 - 4.30 (m, 3H), 4.29 - 4.13 (m, 6H), 4.11 - 3.93 (m, 3H), 3.84 (s, 3H), 3.80 (s, 3H), 3.76 (s, 3H), 3.47 - 3.35 (m, 5H).

$^{13}$C NMR (100 MHz, $CDCl_3$): δ = 157.8, 156.3, 155.0, 148.7, 148.7, 148.5, 147.4, 147.3, 147.3, 147.2, 140.9, 139.3, 139.0, 133.0, 132.9, 132.7, 132.4, 131.8, 131.8, 131.4, 130.8, 130.5, 129.4, 126.8, 120.0, 119.7, 117.3, 116.8, 116.2, 114.9, 114.9, 114.5, 114.4, 112.3, 111.4, 110.4, 67.3, 67.0, 66.5, 65.9, 65.6, 65.2, 56.7, 56.4, 56.3, 36.4, 36.3, 35.1, 35.0, 31.1.

$^{129}$Xe NMR (5 bars, $CDCl_3$): illustrated in figure 1.

RX: illustrated in figure 2.

HRMS-ESI calculated for $C_{50}H_{47}NO_9Na$ [M+Na$^+$] 828.3149, found 828.3162.

Melting point: 257-259 °C.

**Example 2: Alkylation of the nitrogen of 1N-cryptophane**

**Cryptophane 13**

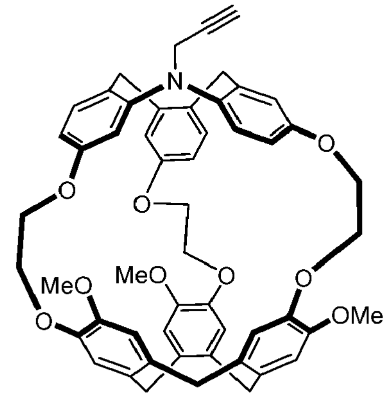

NaH (7.5 mg, 0.186 mmol) was added to a solution of **12** (100 mg, 0.124 mmol) in dry DMF (3 mL) at 0°C and the mixture was stirred for 30 min under nitrogen atmosphere. Propargyl bromide (18 μL, 0.186 mmol) was added and the solution was stirred at 80°C for 3h. The reaction was quenched with a saturated solution of NH4Cl, extracted with ethyl acetate, washed with brine, dried over $MgSO_4$, filtered and concentrated under vacuum. Purification over silica gel ($DCM/Et_2O$ 10/0 to 80/20) afforded the product as a light yellow powder (32 mg, 31%).
$^1H$ NMR (400 MHz, $CDCl_3$): δ = 7.38 (d, J = 8.8 Hz, 1H), 7.14 (d, J = 8.5 Hz, 1H), 7.05 (d, J = 8.4 Hz, 1H), 7.03 (d, J = 2.5 Hz, 1H), 6.81 (d, J = 2.9 Hz, 1H), 6.75 (d, J = 2.6 Hz, 1H), 6.67 (s, 1H), 6.66 (d, J = 1.9 Hz, 2H), 6.63 (s, 1H), 6.61 (d, J = 3.7 Hz, 2H), 6.45 - 6.39 (m, 2H), 6.34 (dd, J = 8.4, 2.6 Hz, 1H), 5.09 (dd, J = 12.2, 10.7 Hz, 2H), 4.60 (dd, J = 13.7, 3.7 Hz, 3H), 4.40 - 4.30 (m, 3H), 4.31 - 4.15 (m, 6H), 4.11 (dd, J = 5.5, 2.4 Hz, 2H), 4.03 (m, 3H), 3.81 (s, 3H), 3.79 (s, 3H), 3.79 (s, 3H), 3.38 (d, J = 13.8 Hz, 3H), 3.30 (d, J = 13.5 Hz, 2H), 2.13 (t, J = 2.4 Hz, 1H).
$^{13}C$ NMR (100 MHz, $CDCl_3$): δ = 157.1, 156.2, 156.1, 150.0, 148.7, 148.6, 148.5, 147.5, 147.4, 147.3, 143.81, 141.84, 141.6, 134.5, 132.87, 132.86, 132.83, 132.4, 132.1, 132.09, 132.06, 130.8, 130.6, 128.0, 119.5, 119.3, 118.1, 117.0, 116.6, 116.4, 114.7, 114.6, 114.5, 112.3, 112.1, 111.4, 80.9, 71.6, 67.1, 66.9, 66.8, 66.0, 65.8, 65.6, 65.4, 56.6, 56.5, 56.4, 47.1, 36.4, 35.0, 34.9, 15.4.
HRMS-ESI calculated for $C_{53}H_{49}NO_9Na$ [M+Na] 866.3305, found 866.3312
Melting point: 274-276°C
Another example of N-alkylation has been performed as follows:

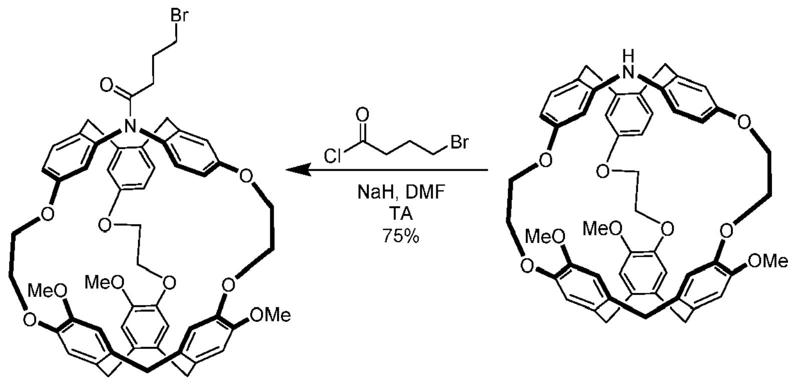


**Example 3: Synthesis of OBn-CTV1N and preparation of a O-protected aza-cryptophane 5-(Benzyloxy)-2-bromo-4-methoxybenzaldehyde 15**

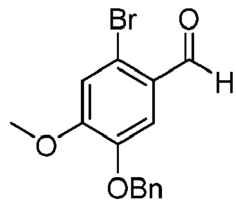

Obtained according to the procedure described by Xie et al. (Bioorganic Med. Chem. 2019, 27 (13), 2764-2770).

**N'-[(Z)-[5-(benzyloxy)-2-bromo-4-methoxyphenyl]methylidene]-4-methylbenzene-1-sulfonohydrazide 16**

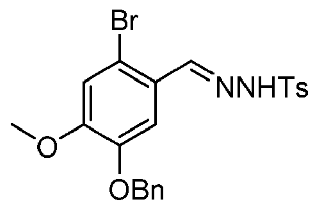

Prepared from **15** (2.5 g, 7.78 mmol), following general procedure A, the expected compound was obtained as a white powder (3.534 g, 93%).
**$^1$H NMR** (400 MHz, $CDCl_3$): δ = 7.98 (s, 1H), 7.79 (d, J = 8.3 Hz, 2H), 7.72 (s, 1H), 7.48-7.45 (m, 3H), 7.43-7.35 (m, 3H), 7.27-7.25 (m, 2H), 6.95 (s, 1H), 5.16 (s, 2H), 3.87 (s, 3H), 2.41 (s, 3H).
**$^{13}$C NMR** (100 MHz, $CDCl_3$): δ = 152.3, 147.9, 146.3, 144.4, 136.5, 135.4, 129.8 (2C), 128.9 (2C), 128.3, 128.1 (2C), 127.6 (2C), 124.4, 116.2, 115.4, 111.4, 71.1, 56.4, 21.8.
**Melting Point:** 175-177°C
**HRMS-ESI** calculated for $C_{22}H_{22}BrN_2O_4S$ [M+H$^+$] 489.0485, found 489.0484

**1-(Benzyloxy)-4-bromo-2-methoxy-5-[(3-methoxyphenyl)methyl]benzene 17**

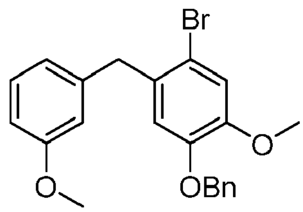

Prepared from **16** (850 mg, 1.73 mmol) and 3-methoxyphenylboronic acid (317 mg, 2.08 mmol) following general procedure B, the expected product was obtained as a white powder (600 mg, 84%).
**$^1$H NMR** (400 MHz, $CDCl_3$): δ = 7.35 - 7.28 (m, 5H), 7.21 (t, J = 7.9 Hz, 1H), 7.07 (s, 1H), 6.78 (dd, J = 8.1, 2.1 Hz, 1H), 6.69 (m, 2H), 6.67 (s, 1H), 5.05 (s, 2H), 3.97 (s, 2H), 3.87 (s, 3H), 3.77 (s, 3H).
**$^{13}$C NMR** (100 MHz, $CDCl_3$): δ = 159.8, 148.8, 147.4, 141.4, 136.7, 132.1, 129.5, 128.6 (2C), 128.0, 127.5 (2C), 121.3, 116.5, 116.0, 115.2, 114.7, 111.6, 71.1, 56.3, 55.2, 41.3.
**Melting Point:** 80-82°C

**4-(Benzyloxy)-N-(2-{[(tert-butyldimethylsilyl)oxy]methyl}-4-methoxyphenyl)-5-methoxy-2-[(3-methoxyphenyl)methyl]aniline 18**

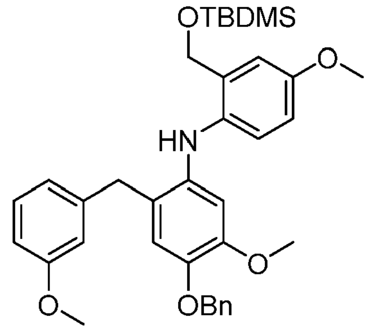

**17** (600 mg, 1.45 mmol), **4** (388 mg, 1.45 mmol), palladium acetate (33 mg, 0.145 mmol), Binap (181 mg, 0.2 mmol), cesium carbonate (1.41 g, 4.35 mmol) were added in toluene (15 mL) under nitrogen atmosphere and heated to reflux for 3h. The mixture was filtrated through a pad of celite using ethyl acetate as the eluent. The solvent was removed under vacuum and purification over silica gel (Cyclohexane/$Et_2O$ 90/10) afforded the product as an orange oil. (539 mg, 62%).
$^1H$ NMR (400 MHz, $CDCl_3$): δ = 7.39 - 7.27 (m, 5H), 7.19 - 7.14 (t, J = 7.9 Hz, 1H), 6.86 (d, J = 2.9 Hz, 1H), 6.82 (d, J = 8.7 Hz, 1H), 6.76 - 6.70 (m, 2H), 6.68 (d, J = 7.5 Hz, 1H), 6.66 (s, 1H), 6.64 - 6.61 (m, 1H), 6.60 (s, 1H), 5.75 (s, 1H), 5.04 (s, 2H), 4.54 (s, 2H), 3.78 (s, 2H), 3.77 (d, 3H), 3.75 (s, 3H), 3.72 (s, 3H).
$^{13}C$ NMR (100 MHz, $CDCl_3$): δ = 159.9, 153.8, 149.2, 143.1, 141.9, 137.6, 137.1, 136.4, 131.0, 129.6, 128.6 (2C), 127.8, 127.7 (2C), 123.2, 121.2, 118.7, 117.9, 114.4, 114.2, 113.3, 111.8, 105.2, 72.0, 63.9, 56.2, 55.8, 55.2, 37.2, 26.0 (3C), 18.4, -5.1 (2C).

**(2-{[4-(Benzyloxy)-5-methoxy-2-[(3-methoxyphenyl)methyl]phenyl]amino}-5-methoxyphenyl)methanol 19**

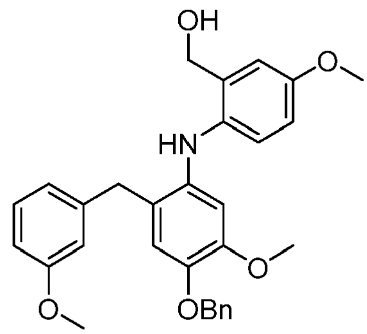

TBAF (1M in THF, 1.33 mL, 1.33 mmol) was added at 0°C to a solution of **18** (535 mg, 0.89 mmol) in THF (20 mL) and the mixture was stirred at room temperature until completion of the reaction. The solution was then quenched with saturated $NaHCO_3$, extracted with ethyl acetate, washed with brine, dried over $MgSO_4$ and concentrated under vacuum. Purification over silica gel (Cyclohexane/$Et_2O$ 5/5) afforded the product as a light yellow powder. (405 mg, 94%).
$^1H$ NMR (400 MHz, $CDCl_3$): δ = 7.44 - 7.40 (m, 2H), 7.39 - 7.34 (m, 2H), 7.31 (m, 1H), 7.18 (t, J = 7.9 Hz, 1H), 6.93 (d, J = 8.6 Hz, 1H), 6.80 (d, J = 2.8 Hz, 1H), 6.78 - 6.74 (m, 3H), 6.70 (d, J = 7.6 Hz, 1H), 6.67 (d, J = 1.8 Hz, 1H), 6.63 (s, 1H), 5.83 (s, 1H), 5.09 (s, 2H), 4.45 (s, 2H), 3.82 (s, 2H), 3.77 (s, 3H), 3.76 (s, 3H), 3.72 (s, 3H), 1.29 (s, 1H).
$^{13}C$ NMR (100 MHz, $CDCl_3$): δ = 159.8, 153.9, 149.3, 142.7, 141.8, 137.6, 137.1, 136.6, 130.7, 129.5, 128.5 (2C), 127.8, 127.7 (2C), 122.2, 121.1, 119.5, 118.5, 115.1, 114.3, 114.1, 111.7, 104.5, 72.1, 63.9, 56.1, 55.7, 55.2, 37.6.
**Melting Point:** 103-105°C

**3-Benzyloxy-2,7,12-trimethoxy-10,15-dihydro-*5H*-tribenzo[*b,e,h*]azonine 20**

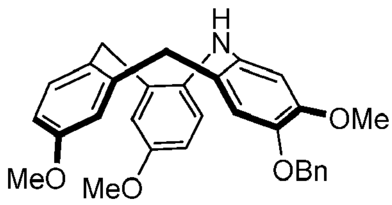

Phosphorus pentoxide (37 mg, 0.13 mmol) was added to a solution of **19** (50 mg, 0.1 mmol) in acetonitrile (20 mL) at 0°C and then the solution was heated to 60°C for 1h. The reaction was quenched with water, extracted with ethyl acetate, washed with brine, dried over $MgSO_4$ and concentrated under vacuum. Purification over silica gel (cyclohexane/$Et_2O$ 8/2) afforded the product as a light pink powder (36 mg, 78%).
$^1H$ NMR (400 MHz, $CDCl_3$): $\delta$ = 7.35 (m, 2H), 7.30 (m, 2H), 7.24 (s, 1H), 7.04 (d, J = 7.08 Hz, 1H), 6.74 (d, J = 8.7 Hz, 1H), 6.68 - 6.64 (m, 3H), 6.50 - 6.45 (m, 2H), 6.35 (s, 1H), 5.55 (s, 1H), 4.94 (s, 2H), 3.83 (s, 3H), 3.80 (s, 3H), 3.79 (s, 2H), 3.68 (s, 3H), 3.67 (s, 2H).
$^{13}C$ NMR (100 MHz, $CDCl_3$): $\delta$ = 158.4, 154.6, 149.4, 142.7, 141.0, 138.0, 137.5 (2C), 137.0, 132.1, 131.0, 130.1, 128.5 (2C), 127.8 (2C), 120.8, 119.3, 118.0, 117.3, 116.6, 113.1, 110.6, 103.8, 72.1, 56.1, 55.5, 55.3, 35.5, 29.8.
Melting point: 93-95°C

The corresponding aza-cryptophane is obtained similarly to what has been described above regarding example 1.

**Example 3: Synthesis of 2N-cryptophane of the invention**

**4-Methoxy-N-(3-methoxyphenyl)-2-nitroaniline 21**

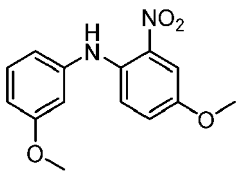

3-bromoanisole (1.5 g, 8.02 mmol), 4-methoxy-2-nitroaniline (1.35 g, 8.02 mmol), palladium acetate (180 mg, 0.80 mmol), 2,2'-bis(diphenylphosphino)-1,1'-binaphthalene (998 mg, 1.60 mmol), cesium carbonate (9.1 g, 28.07 mmol) were added in anhydrous toluene (40 mL) placed under nitrogen and heated to reflux for 3h. The mixture was filtrated through a pad of celite using ethyl acetate as eluent. The solvent was removed under vacuum and purification over silica gel (cyclohexane/$Et_2O$ 95/5) afforded the product as a dark red oil. (2.13 g, 97%).
$^1H$ NMR (400 MHz, $CDCl_3$): $\delta$ = 9.28 (s, 1H), 7.60 (d, J = 3.0 Hz, 1H), 7.31 - 7.24 (m, 2H), 7.06 (dd, J = 9.4, 3.0 Hz, 1H), 6.82 (dd, J = 7.9, 1.8 Hz, 1H), 6.77 (t, J = 2.2 Hz, 1H), 6.71 (dd, J = 8.1, 2.1 Hz, 1H), 3.81 (s, 3H), 3.80 (s, 3H).
$^{13}C$ NMR (100 MHz, $CDCl_3$): $\delta$ = 160.8, 151.4, 140.7, 137.7, 133.2, 130.5, 126.2, 118.5, 115.6, 110.5, 109.1, 107.0, 55.9, 55.5.

**4-Methoxy-N1-(3-methoxyphenyl)benzene-1,2-diamine 22**

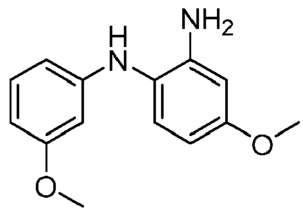

**21** (2 g, 7.3 mmol) and $SnCl_2$ (16.4 g, 73 mmol) were dissolved in a 1/1 mixture acetonitrile/water (40 mL) and the solution was stirred at 80°C overnight. After completion of the reaction, a saturated solution of potassium carbonate

and a 2M solution of sodium tartrate were added, the solution was stirred for two hours and extracted three times with ethyl acetate. The organic layers were combined, washed with brine, dried over $MgSO_4$, filtered and concentrated under vacuum. The expected product was obtained without purification as a light yellow powder (1.77 g, 99%).
$^1H$ NMR (400 MHz, $CDCl_3$): δ = 7.08 (t, J = 8.1 Hz, 1H), 7.00 (d, J = 8.5 Hz, 1H), 6.36 - 6.29 (m, 3H), 6.25 (ddd, J = 8.1, 2.1, 0.7 Hz, 1H), 6.18 (t, J = 2.3 Hz, 1H), 5.03 (s, 1H), 3.86 (s, J = 9.9 Hz, 1H), 3.78 (s, J = 4.9 Hz, 3H), 3.74 (s, J = 4.0 Hz, 3H).
$^{13}C$ NMR (100 MHz, $CDCl_3$): δ= 160.9, 158.8, 148.4, 144.9, 130.2, 128.8, 120.6, 106.9, 104.2, 103.7, 101.3, 100.0, 55.4, 55.2.

**N2-(2-{[(Tert-butyldimethylsilyl)oxy]methyl}-4-methoxyphenyl)-4-methoxy-N1-(3-methoxyphenyl)benzene-1,2-diamine 23**

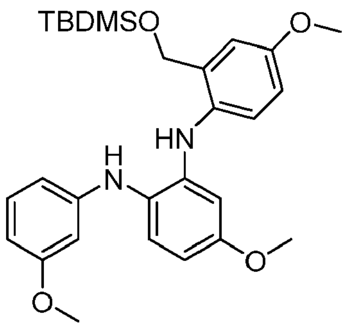

**4** (1.42 g, 5.8 mmol), **22** (1.93 g, 5.8 mmol), palladium acetate (130 mg, 0.58 mmol), 2,2'-bis(diphenylphosphino)-1,1'-binaphthalene (722 mg, 1.16 mmol) and cesium carbonate (6.6 g, 20.3 mmol) were added in anhydrous toluene (50 mL) placed under nitrogen and heated to reflux for 3h. The mixture was filtrated through a pad of celite using ethyl acetate as eluent. The solvent was removed under vacuum and purification over silica gel (cyclohexane/$Et_2O$ 9/1 to 8/2) afforded the product as a green oil. (1.79 g, 63%).
$^1H$ NMR (400 MHz, $CDCl_3$): δ = 7.19 (d, *J* = 8.7 Hz, 1H), 7.07 (ddd, *J* = 12.6, 8.1, 4.6 Hz, 2H), 6.94 (d, *J* = 3.0 Hz, 1H), 6.78 (dd, *J* = 8.7, 3.0 Hz, 1H), 6.44 (d, *J* = 2.8 Hz, 1H), 6.36 - 6.29 (m, 4H), 6.25 (t, *J* = 2.3 Hz, 1H), 5.05 (s, 1H), 4.58 (s, 2H), 3.80 (s, *J* = 3.2 Hz, 3H), 3.73 (s, 3H), 3.71 (s, 3H), 0.84 (s, *J* = 2.9 Hz, 9H), -0.02 (s, *J* = 3.1 Hz, 6H).
$^{13}C$ NMR (100 MHz, $CDCl_3$): δ = 160.9, 158.5, 155.9, 148.3, 143.5, 135.9, 133.1, 130.1, 127.8, 124.1, 122.2, 113.8, 113.3, 107.7, 104.3, 103.8, 100.7, 100.0, 63.2, 55.6, 55.4, 55.2, 25.9 (3C), 18.4, -5.2 (2C).

**[5-Methoxy-2-({5-methoxy-2-[(3-methoxyphenyl)amino]phenyl}amino)phenyl]methanol 24**

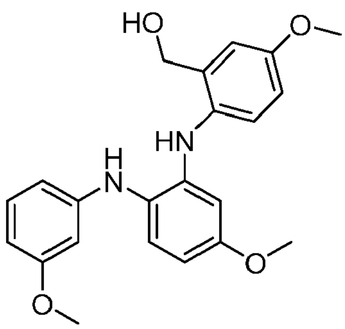

TBAF (1M in THF, 7.2 mL, 7.2 mmol) was added at 0°C to a solution of **23** (1.79 g, 3.62 mmol) in THF (30 mL) and the mixture was stirred at room temperature until completion of the reaction. The reaction mixture was quenched with a saturated aqueous solution of $NaHCO_3$, extracted three times with ethyl acetate. The organic layers were combined, washed with brine, dried over $MgSO_4$, filtered and concentrated under vacuum. Purification over silica gel (cyclohexane/$Et_2O$ 5/5) afforded the product as a light yellow oil. (1.034 g, 96%).
$^1H$ NMR (400 MHz, $CDCl_3$): δ = 7.23 (d, *J* = 8.6 Hz, 1H), 7.11 - 7.05 (m, 2H), 6.85 (d, *J* = 2.9 Hz, 1H), 6.81 (dd, *J* = 8.6,

3.0 Hz, 1H), 6.46 (d, *J* = 2.6 Hz, 2H), 6.37 - 6.28 (m, 3H), 6.23 (t, *J*= 2.2 Hz, 1H), 5.20 (s, 1H), 4.48 (s, 2H), 3.78 (s, 3H), 3.72 (s, 3H), 3.71 (s, 3H), 1.99 (s, 1H). $^{13}$C NMR (100 MHz, $CDCl_3$): δ = 160.9, 158.7, 155.9, 148.4, 143.6, 135.0, 133.7, 130.1, 128.6, 124.2, 121.5, 114.8, 114.0, 107.2, 104.0, 103.6, 100.2, 100.0, 63.4, 55.6, 55.4, 55.2.

**2,7,12-Trimethoxy-10,15-dihydro-5H-tribenzo[b,e,h][1,4]diazonine 25**

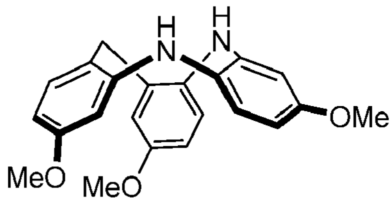

Perchloric acid (60% in water, 0.160 mL) was added dropwise to a solution of **24** (100 mg, 0.264 mmol) in acetonitrile (53mL) at 0°C and the solution was heated to 60°C for 1h. The reaction mixture was quenched with water, extracted three times with ethyl acetate. The organic layers were combined, washed with brine, dried over $MgSO_4$, filtered and concentrated under vacuum. Purification over silica gel (cyclohexane/$Et_2O$ 9/1 to 8/2) afforded the product as a white powder (58 mg, 61%).
$^1$H NMR (400 MHz, $CDCl_3$): δ = 6.95 (dd, *J* = 8.3, 7.3 Hz, 2H), 6.86 (d, *J* = 8.6 Hz, 1H), 6.66 (dd, *J* = 8.5, 3.0 Hz, 1H), 6.55 (d, *J* = 2.9 Hz, 1H), 6.39 (td, *J* = 8.6, 2.7 Hz, 2H), 6.34 (d, *J* = 2.5 Hz, 1H), 6.31 (d, *J* = 2.8 Hz, 1H), 5.38 (s, 1H), 4.79 (s, 1H), 3.76 (s, 3H), 3.73 (s, 6H), 3.63 (s, 2H).
$^{13}$C NMR (100 MHz, $CDCl_3$): δ = 159.1, 157.9, 156.2, 145.4, 142.9, 136.7, 136.2, 132.5, 128.4, 126.6, 124.5, 122.3, 116.7, 111.6, 107.1, 106.6, 106.1, 106.0, 55.5 (2C), 55.3, 34.9.
Melting point: 166-168°C

**10,15-Dihydro-5H-tribenzo[b,e,h][1,4]diazonine-2,7,12-triol 26**

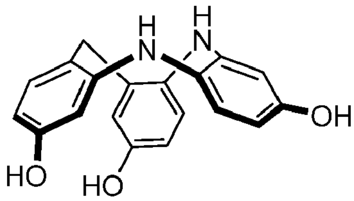

BBr3 (1M in DCM, 4.6 mL, 4.6 mmol) was added to a solution of **25** (475 mg, 1.3 mmol) in dichloromethane (7 mL) at 0°C and the reaction mixture was stirred at room temperature until completion of reaction. The reaction was quenched at 0°C with water, extracted three times with ethyl acetate. The organic layers were combined, washed with brine, dried over $MgSO_4$, filtered and concentrated under vacuum. Purification over silica gel (cyclohexane/$Et_2O$ 5/5 to 7/3) afforded the product as a light brown powder (295 mg, 70%).
$^1$H NMR (400 MHz, Acetone-$d_6$): δ = 7.94 (s, 1H), 7.92 (s, 1H), 7.71 (s, 1H), 6.88 (d, J = 8.1 Hz, 1H), 6.76 (dd, J = 10.6, 8.4 Hz, 2H), 6.51 (dd, J = 8.4, 2.9 Hz, 1H), 6.42 (m, 3H), 6.33 (d, J = 2.7 Hz, 1H), 6.28 (dd, J = 8.1, 2.5 Hz, 1H), 6.22 (dd, J = 8.4, 2.8 Hz, 1H), 5.49 (s, 1H), 3.58 (s, 2H).
$^{13}$C NMR (100 MHz, Acetone-$D_6$): δ = 158.1, 157.1, 154.5, 148.7, 146.0, 137.8, 136.5, 133.2, 131.3, 126.6, 124.9, 123.4, 118.8, 114.4, 108.9, 108.7, 108.4, 107.4, 35.9.

**(((((10,15-Dihydro-5H-tribenzo[b,e,h][1,4]diazonine-2,7,12-triyl)tris(oxy))tris(ethane-2,1-diyl))tris(oxy))tris(3-methoxybenzene-4,1-diyl))trimethanol 27**

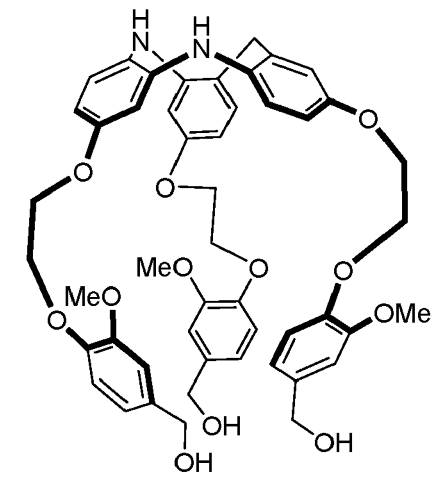

**10** (1.67 g, 5.4 mmol) and cesium carbonate (1.76 g, 5.4 mmol) were added to a solution of 26 (290 mg, 0.905 mmol) in dry DMF (14 mL) placed under nitrogen. The mixture was stirred overnight at 80°C, and allowed to reach room temperature. The reaction was quenched with water and the product was extracted three times with ethyl acetate. The organic layers were combined, washed with brine, dried over $MgSO_4$, filtered and concentrated under vacuum. Purification over silica gel (AcOEt/MeOH, 100/0 to 96/4) afforded the product as a pink powder (485 mg, 62%).
$^1$H NMR (400 MHz, DMSO): δ = 7.58 (s, 1H), 7.03 (d, J = 8.4 Hz, 1H), 6.99 - 6.94 (m, 5H), 6.86 - 6.83 (m, 3H), 6.80 (dd, J = 8.8, 3.5 Hz, 3H), 6.68 (dd, J = 8.6, 2.9 Hz, 1H), 6.53 (d, J = 2.5 Hz, 1H), 6.48 (d, J = 2.8 Hz, 1H), 6.43 - 6.39 (m, 2H), 6.36 (dd, J = 8.6, 2.8 Hz, 1H), 6.06 (s, 1H), 4.45 (m, 6H), 4.26 (m, 4H), 4.24 - 4.19 (m, 6H), 4.14 - 4.12 (m, 2H), 3.78 (s, 3H), 3.77 (s, 3H), 3.75 (s, 3H), 3.55 (s, 2H).
Melting point: 99-101°C.

The corresponding aza-cryptophane has been obtained as follows, similarly to what has been described above regarding example 1:

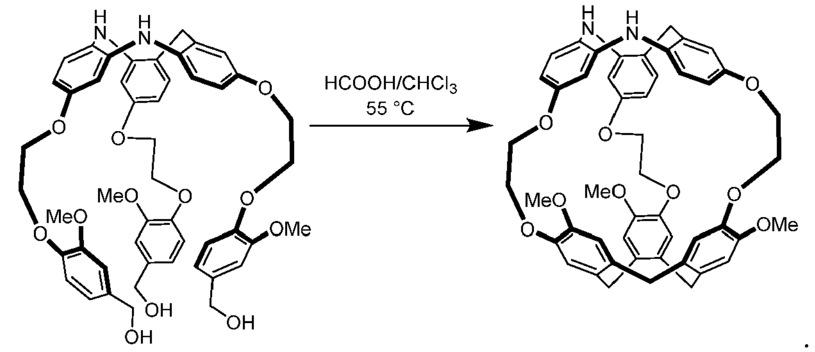


**Example 4: Comparison of the solubility of aza-cryptophanes of the invention VS conventional cryptophanes outside the invention**

Experimental solubility of cryptophane A (conventional cryptophane outside the invention):

DMSO : 25 mg/mL or 27.93 µmol/mL (corresponds to the solubility limit)
$CHCl_3$: not tested
MeCN: very poorly soluble.

Experimental solubility of Cryptophane-1N :

DMSO: 59.5 mg/mL or 73.8 μmol/mL (solubility limit not reached)
$CHCl_3$: 8.27 μmol/mL (solubility limit not reached)
MeCN: 2.23 μmol/mL (solubility limit not reached)

**Example 5: Synthesis of a N-protected 2N-cryptophane**

A N-protected 2N-cryptophane has been obtained as follows:

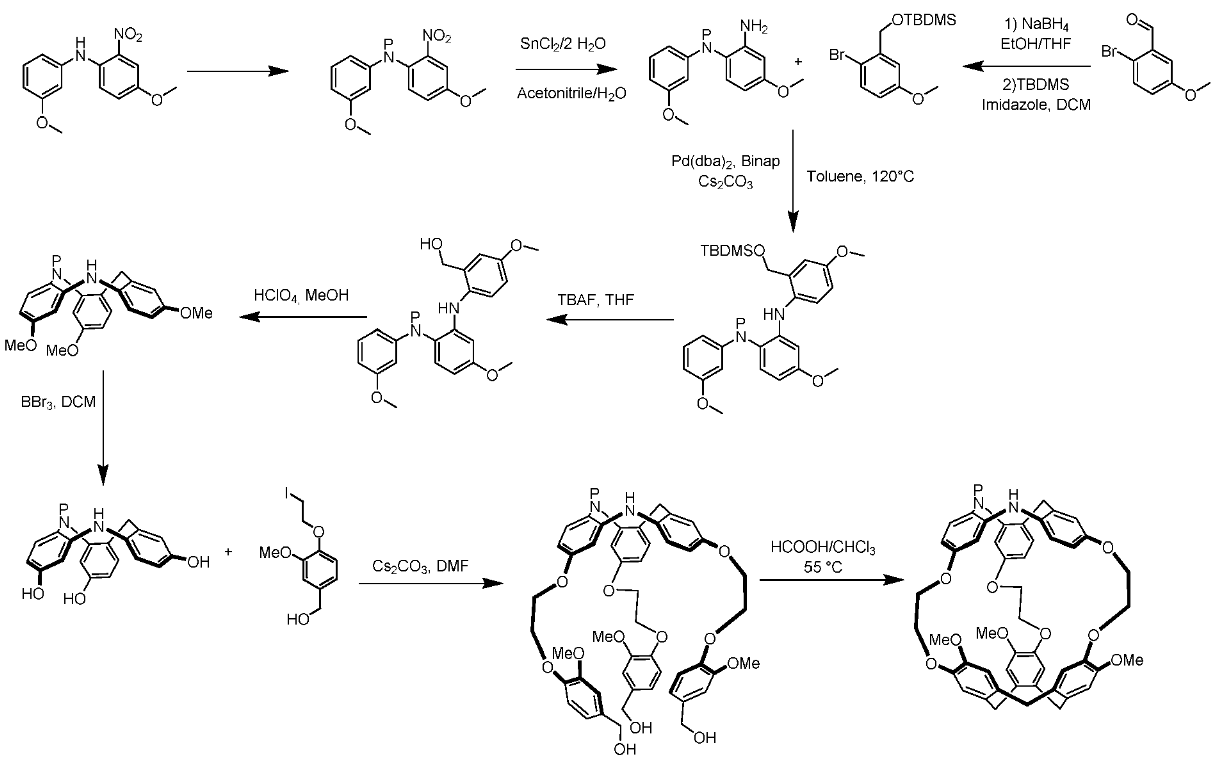


wherein P is a benzyl, *p*-methoxybenzyl, 2-tetrahydropyranyl (THP), a carbamate, for example Boc, Fmoc, Troc, Cbz, a silyl, in particular *tert*-butyldimethylsilyl (TBS or TBMS), trimethylsilyl (TMS), triisopropyl silyl (TIPS), triethylsilyl (TES), *tert*-butyldiphenylsilyl (TBDPS), or 2-(trimethylsilyl)ethoxymethyl (SEM), or an acyl group, in particular an acetyl.

**Example 6: synthesis of an aza-cryptophane with n1= n2=n3=0**

An aza-cryptophane with n1= n2=n3=0 is obtained as follows:

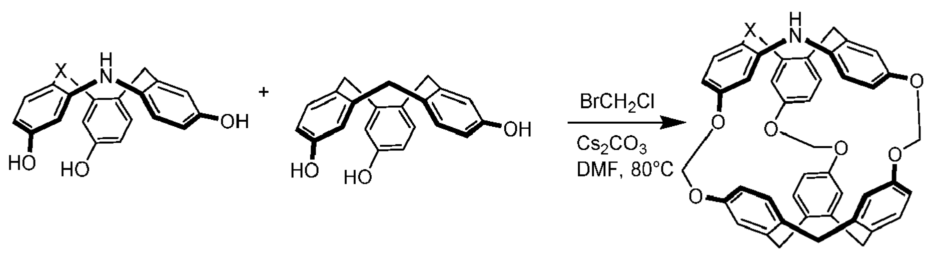

,

wherein X is CH2 or NH or NP, wherein P is a benzyl, *p*-methoxybenzyl, 2-tetrahydropyranyl (THP), a carbamate, for example Boc, Fmoc, Troc, Cbz, a silyl, in particular *tert*-butyldimethylsilyl (TBS or TBMS), trimethylsilyl (TMS), triisopropylsilyl (TIPS), triethylsilyl (TES), *tert*-butyldiphenylsilyl (TBDPS), or 2-(trimethylsilyl)ethoxymethyl (SEM), or an acyl group, in particular an acetyl.

An aza-cryptophane with n1= n2=n3=0 can also be obtained as follows:

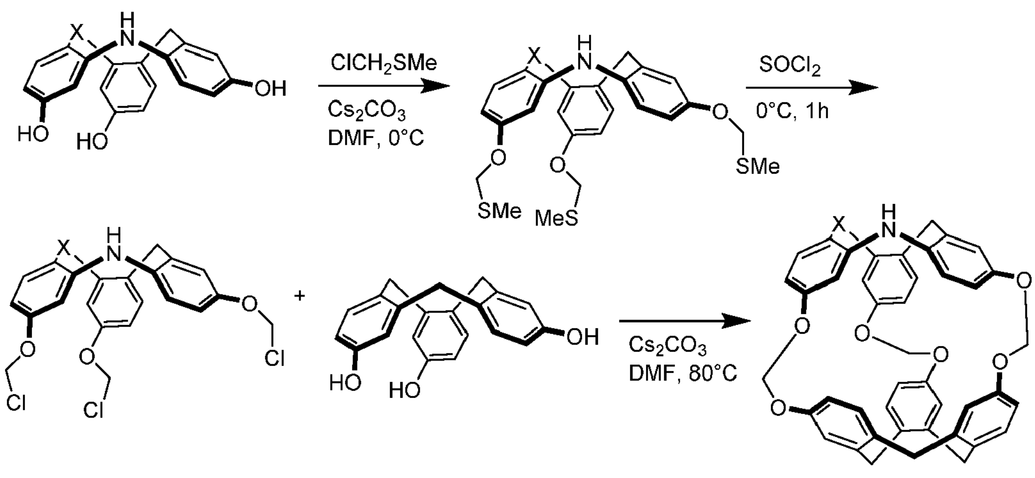


wherein X is CH2 or NH or NP, wherein P is a benzyl, *p*-methoxybenzyl, 2-tetrahydropyranyl (THP), a carbamate, for example Boc, Fmoc, Troc, Cbz, a silyl, in particular *tert*-butyldimethylsilyl (TBS or TBMS), trimethylsilyl (TMS), triisopropyl silyl (TIPS), triethylsilyl (TES), *tert*-butyldiphenylsilyl (TBDPS), or 2-(trimethylsilyl)ethoxymethyl (SEM), or an acyl group, in particular an acetyl.

**Example 7: synthesis of an 3N-cryptophane**

A 3N aza-cryptophane is obtained as follows:

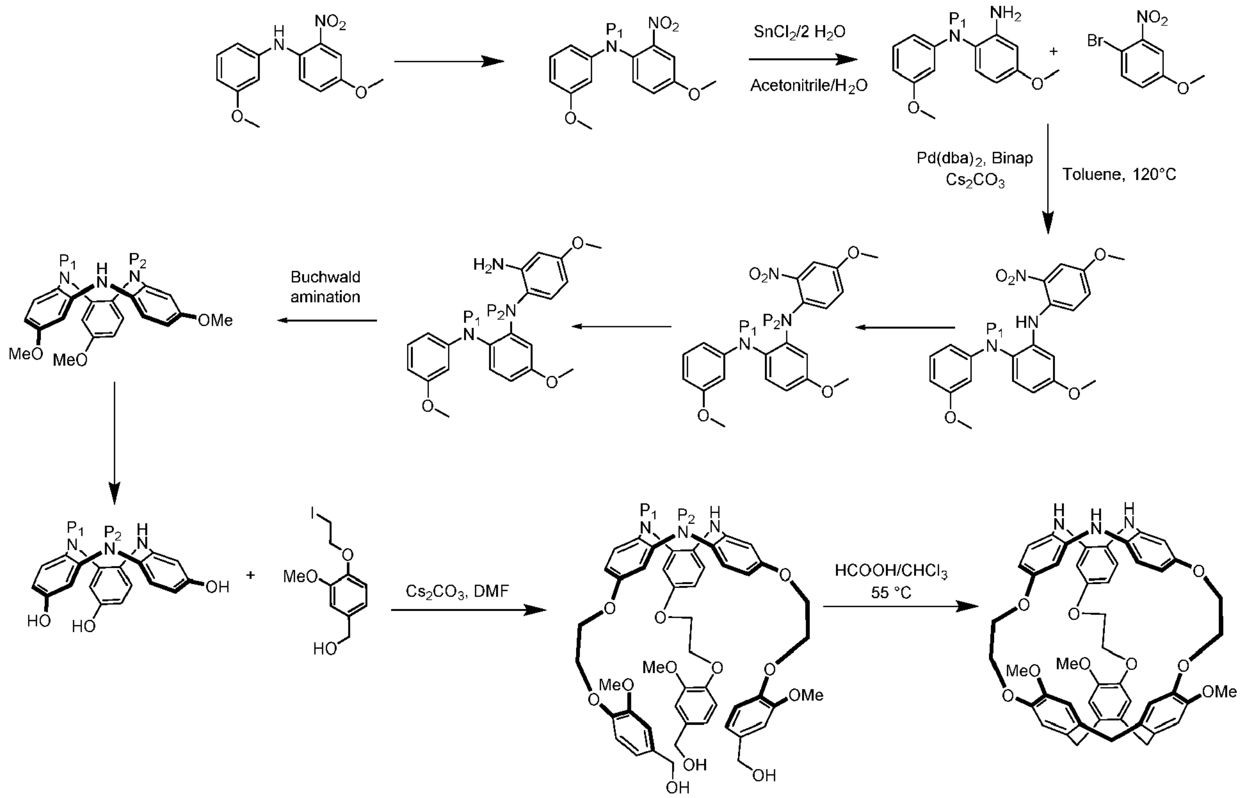


wherein $P_1$ and $P_2$ are chosen from benzyl, *p*-methoxybenzyl, 2-tetrahydropyranyl (THP), carbamate, for example Boc, Fmoc, Troc, Cbz, silyl, in particular *tert*-butyldimethylsilyl (TBS or TBMS), trimethylsilyl (TMS), triisopropyl silyl (TIPS), triethylsilyl (TES), *tert*-butyldiphenylsilyl (TBDPS), or 2-(trimethylsilyl)ethoxymethyl (SEM), and acyl groups, in particular an acetyl.

**Example 8: selective dealkylation of a compound of formula (I) with $Z_1$, $Z_2$ or $Z_3$ being - O-$C_1$-$C_6$ alkyl, notably OMe, to obtain the corresponding compound with $Z_1$, $Z_2$ or $Z_3$ being -OH**

Iodotrimethylsilane (0.6 eq.) was added in one portion to a stirred solution of cryptophane (1 eq.) in dichloromethane. The solution was stirred in the dark for 16 h under nitrogen at room temperature. Dichloromethane was added and the solution was quenched with hydrochloric acid (1M), the organic layer was washed with water, dried over $MgSO_4$, filtered and concentrated under vacuum. Purification on alumina neutral gel of the crude mixture afforded the expected product.

**Example 9: Nanoemulsions comprising a aza-cryptophane of the invention**

Nanoemulsions comprising the 1N-cryptophane **12** (Cr1N) of the invention and control nanoemulsions comprising cryptophane A (CrA, outside the invention) have been preprared as follows.

Protocol:

Nanoemulsion formulation was adapted from spontaneous nano-emulsification method previously described by Séguy et al. (Pharmaceutics. 2020, 12, 1141;). Rearding Cr1N, the anhydrous phase, composed of oil (Labrafac® WL 1349), surfactant (Kolliphor® HS 15) and solubilizer (Transcutol® HP), and CrlN **12** were heated at 70°C under gentle magnetic stirring (250 rpm) and cooled down at 25°C.

Similarly, for CrA, the anhydrous phase, composed of oil (Labrafac® WL 1349), surfactant (Kolliphor® HS 15) and solubilizer (Transcutol® HP), was heated at 70°C under gentle magnetic stirring (250 rpm). 160µL of a saturated solution of CrA in DMSO, and the anhydrous phase was heated at 70°C for 15 minutes under gentle magnetic stirring, then allowed to reach 25°C.

In both cases, when the anhydrous mixture reached this temperature, the magnetic stirring was increased from 250 rpm to 750 rpm and the aqueous phase (HPLC grade water, 25°C) was suddenly added, leading to spontaneous emulsification. After the addition of water, the stirring was maintained for 15 minutes at room temperature. Then, the formulation was filtered through 0.2 µm regenerated cellulose syringe filters (Minisart® Syringe Filter, Sartorius, Goettingen, Germany).

Physico-chemical characterization of nanoemulsions:

Dynamic light scattering (DLS) was used to determine the average hydrodynamic diameter, the polydispersity index (PDI) and the diameter distribution by volume of the nanoemulsions using a Zetasizer ultra apparatus (Malvern Instruments, Worcestershire, UK) equipped with a 633 nm laser at a fixed scattering angle of 173°. The temperature of the cell was kept constant at 25°C.

The nanoemulsions were diluted 1/100 (v/v) in NaCl 1 mM in order to assure an appropriate scattered intensity on the detector before measurements. Measurements were performed in triplicate.

Potential zeta measurements:

Zeta potential analyses were realized, after filtration and 1/100 dilution in NaCl 1 mM, using a Zetasizer ultra apparatus equipped with DTS 1070 cell. All measurements were performed in triplicate at 25°C, with a dielectric constant of 78.5, a refractive index of 1.33, a viscosity of 0.8872 cP and a cell voltage of 150 V. The zeta potential was calculated from the electrophoretic mobility using the Smoluchowski equation.

Calibration curve:

Cryptophanes concentration were defined using spectrophotometry (Infinite M200, Tecan, Männedorf, Switzerland) by determining absorbance spectrum of Crytophane in solution in MeOH.

Dosage :

The encapsulation efficiency (EE) was determined after filtration through 0.2 µm syringe filters (Minisart® Syringe Filter, Sartorius, Goettingen, Germany) to remove unentrapped cryptophanes. Then, these samples were diluted in methanol (1/100, v/v) and the concentration of cryptophane was determined by spectrophotometry at 288 nm. The EE was determined in duplicate and calculated as follows:

$$EE\ (\%)=100 \times \frac{\text{Quantity of API entrapped}}{\text{Total quantity of API added}} \quad [1]$$

The drug loading (DL) was defined as:

$$DL\ (\%)=100 \times \frac{\text{Quantity of API entrapped}}{\text{Total quantity of anhydrous excipients}} \quad [2]$$

**Results**

Cryptophane-1N:

1 batch of nanoemulsions was prepared, using 15 mg of cryptophane-1N (corresponding to a final CrIN concentration of 81.6 μM, see protocol above). The hydrodynamic diameter (ϕ) of the formulated nanoemulsions, the polydispersity index (PDI) and the surface charge (ζ) of the formed objects were characterised (see table 1 below).

Table 1: Physico-chemical properties of empty or Cr1N-charged nanoemulsions

| Load | « Empty » nanoemulsions | | « Loaded » nanoemulsions | | ζ (mV) |
|---|---|---|---|---|---|
| | ϕ(nm) | PDI | ϕ(nm) | PDI | |
| 15.1 mg CrIN (= 18.74 μmol) | 52.9 ± 0.4 | 0.209 ± 0.003 | 47.4 ± 0.6 | 0.150 ± 0.001 | -4.9 ± 0.4 |

The obtained particles have an average hydrodynamic diameter of approximately 47 nm. This diameter is significantly smaller than the one observed for nanoemulsions formulated without organic molecules (i.e. "empty" nanoemulsions), which indicates that cryptophane molecules participate in the stabilisation of nanoemulsions.

In addition, the UV evaluation of the batch was carried out. The obtained UV spectra shows the presence of a signal at 288 nm, which is characteristic of the cage, and not present for "empty" nanoemulsions.

From these results, nanoemulsions appear able to encapsulate the cryptophane molecules.

Cryptophane A:

1 batch of nanoemulsions was prepared, using a saturated solution of cryptophane A in DMSO (53.3 mg in 2.13 mL, see protocol above). The hydrodynamic diameter of the formulated nanoemulsions, the polydispersity index and the surface charge of the formed objects were characterised (see table 2 below). In addition, the UV evaluation of the batch could be carried out.

Table 2: Physico-chemical properties of empty or CrA-charged nanoemulsions

| Load | « Empty » nanoemulsions | | « Loaded » nanoemulsions | | ζ (mV) |
|---|---|---|---|---|---|
| | ϕ(nm) | PDI | ϕ(nm) | PDI | |
| 160 μL of CrA/DMSO solution | 64.8 ± 0.4 | 0.193 ± 0.007 | 60.3 ± 0.8 | 0.144 ± 0.016 | -6.4 ± 0.8 |

Dosing of the effective encapsulated quantity :

A UV calibration curve for each of the compounds was carried out (see protocol above) using a stock solution of CrIN in acetonitrile (1.05 mg/mL) and a stock solution of cryptophane A in DMSO (1.85 mg/mL), solutions subsequently diluted in methanol.

The batch of nanoemulsions loaded with CrIN was diluted 100-fold in methanol. Under these conditions, it was confirmed by DLS that the nanoemulsions were destroyed. The amount of cryptophane in this sample was then determined. The concentration of cryptophane in the sample was 22 μM, i.e. an encapsulation efficiency of 27%.

Similarly, the batch of nanoemulsions loaded with CrA was diluted under the same conditions. The concentration of the sample is 11 μM, an encapsulation efficiency of 49%.

It has been thus demonstrated that, thanks to the high solubility of 1N cryptophane, it is possible to load twice as much 1N cryptophane as cryptophane A into these nanoemulsions, with no need for prior dissolution in DMSO.

Stability over time

Stability monitoring of the batche loaded with CrIN was carried out, making it possible to demonstrate that these nanoemulsions are stable at 4°C for at least 7 days (table 3).

| | $\Phi$ (nm) | PDI | $\zeta$ (mV) |
|---|---|---|---|
| 3 hours | 47.4 ± 0.6 | 0.150 ± 0.001 | -4.9 ± 0.4 |
| 24 hours | 47.9 ± 0.9 | 0.140 ± 0.010 | - |
| 7 days | 48.9 ± 0.4 | 0.148 ± 0.008 | -5.7 ± 0.8 |

In addition, the UV absorbance spectrum of the nanoemulsions charged with CrIN does not evolve over time (from 3 hours to 7 days).

## Claims

1. Compound of following formula (I):

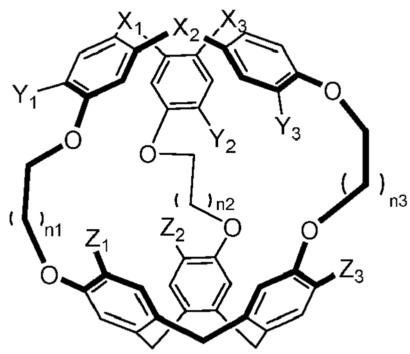

(I)

Wherein:

$X_1$, $X_2$ and $X_3$ are independently selected from $-CH_2-$ and -NR-, at least one of $X_1$, $X_2$ and $X_3$ representing -NR-; R is chosen from:

- H;
- a $C_1$-$C_6$ alkyl;
- a $C_2$-$C_6$ alkenyl;
- a $C_2$-$C_6$ alkynyl;
- a LZ group;
- an *ad hoc* protecting group, in particular a benzyl, *p*-methoxybenzyl, 2-tetrahydropyranyl, a carbamate, for example Boc, Fmoc, Troc, Cbz, a silyl, in particular *tert*-butyldimethylsilyl, trimethylsilyl, triisopropylsilyl, triethylsilyl, *tert*-butyldiphenylsilyl, or 2-(trimethylsilyl)ethoxymethyl, or an acyl group, in particular an acetyl;

$Y_1$, $Y_2$ and $Y_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- a O-L'Z' group;
- OH or a O-P' group, wherein P' is an *ad hoc* protecting group, in particular a benzyl; *p*-methoxybenzyl;

silyl, in particular *tert*-butyldimethylsilyl, trimethylsilyl, triisopropylsilyl, triethylsilyl, *tert*-butyldiphenylsilyl; 2-(trimethylsilyl)ethoxymethyl; allyl; methoxymethyl ; tetrahydropyran or an acyl group, in particular an acetyl;

$Z_1$, $Z_2$ and $Z_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- a O-L"Z" group;
- OH or a O-P" group, wherein P" is an *ad hoc* protecting group, in particular a benzyl; *p*-methoxybenzyl; silyl, in particular *tert*-butyldimethylsilyl, trimethylsilyl, triisopropylsilyl, triethylsilyl, *tert*-butyldiphenylsilyl ; 2-(trimethylsilyl)ethoxymethyl ; allyl; methoxymethyl ; tetrahydropyran or an acyl group, in particular an acetyl;

n1, n2 and n3 are independently chosen from 0, 1, 2 and 3;
L, L' and L" are independently from each other a linker;
Z, Z' and Z" are independently from each other:

- a terminal reactive function or group, in particular able to form a covalent bond with a peptide, a protein, an antibody or a fragment thereof, a carrier, a solid support, a multivalent scaffold; an anchor; a dye or a fluorescent residue; or
- a peptide, a protein, an antibody or a fragment thereof, a carrier, a solid support, a multivalent scaffold; an anchor; a dye or a fluorescent residue;

or a pharmaceutically acceptable salt thereof.

**2.** Compound according to claim 1, wherein:

- $X_1$ is -NR-, and $X_2$ and $X_3$ are -$CH_2$-; or
- $X_1$ and $X_2$ are independently selected from -NR- groups, and $X_3$ is -$CH_2$-; or
- $X_1$, $X_2$ and $X_3$ are independently selected from -NR- groups;

and/or
- $Y_1$, $Y_2$ and $Y_3$ are H;

and/or
- $Z_1$, $Z_2$ and $Z_3$ are H, or a O-$C_1$-$C_6$ alkyl, in particular OMe;

and/or
- n1 = n2 = n3 = 0 or 1, in particular 1.

**3.** Compound according to anyone of preceding claims, wherein:

- $X_1$ is -NR- with R being LZ, $X_2$ and $X_3$ being in particular -$CH_2$- or -NH-; and/or
- $Y_1$ is O-L'Z', $Y_2$ and $Y_3$ being in particular H; and/or
- $Z_1$ is O-L"Z", $Z_2$ and $Z_3$ being in particular a O-$C_1$-$C_6$ alkyl, more particularly OMe.

**4.** Compound according to anyone of preceding claims, wherein L is $(W)_i$-$L_1$-$(F_1$-$L_2)_j$-$(F_2)_k$- and/or L' is $(W')_i$-$L'_1$-$(F'_1$-$L'_2)_j$-$(F'_2)_k$-, wherein:

- W and W' are independently chosen from -C(=O)-, -C(=O)O-, -C(=O)NH-,-C(=S)-O-
- $L_1$, $L_2$, $L'_1$ and $L'_2$ are independently is a $C_1$-$C_{12}$ alkane diyl, a $C_2$-$C_{12}$ alkene diyl or a $C_2$-$C_{12}$ alkyne diyl chain optionally interrupted by one or more heteroatoms, notably selected from an oxygen atom, a sulphur atom or a nitrogen atom, said nitrogen and sulphur atoms being optionally oxidized;
- $F_1$, $F_2$, $F'_1$ and $F'_2$ are independently chosen from -C(=O)-, -C(=O)-C(=O)-, -C(=O)-C(=O)-NH-, -NHC(=O)-C(=O)-, -NHC(=O)-C(=O)-NH-, -C(=O)-C(H)=N-NH-, -NH-C(=O)-C(H)=N-NH-, ester, amide, amine, -$CH_2$-, ether, thioether, imine, succinimide, thio-succinimide, oxime, hydrazone, hydrazonamide,

-C(=O)$CH_2$-NH-, -NH-$CH_2$-C(=O)-, triazole functions or groups;
- i, j, and k are independently 0 or 1.

**5.** Compound according to claim 4, wherein:

- k is 0, Z being in particular a terminal reactive function or group, in particular able to form a covalent bond with a peptide, a protein, a carrier, a solid support, a multivalent scaffold; an anchor; a dye or a fluorescent residue, Z being more particularly a halogen, in particular Cl, Br, I, F, more particularly Br, $C_2$-$C_6$ alkenyl, $C_2$-$C_6$ alkynyl, azido, epoxide, C(=O)H, hemiacetal, C(=O)$R_c$, acetal, $SR_a$, $NH_2$ or NHC(=O)$CH_2$Hal, wherein Hal is a halogen, in particular Cl, Br, I, F, more particularly Br, N-maleimide; or
- k is 1, Z being in particular a peptide, a protein, a carrier, a solid support, a multivalent scaffold; an anchor; a dye or a fluorescent residue.

**6.** Compound according to claim 1, chosen from the following formulae:

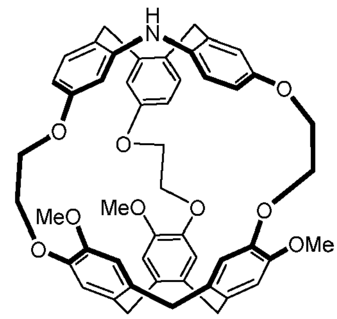
,
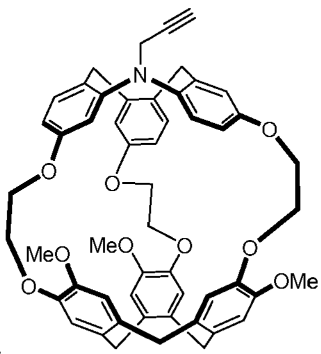
,
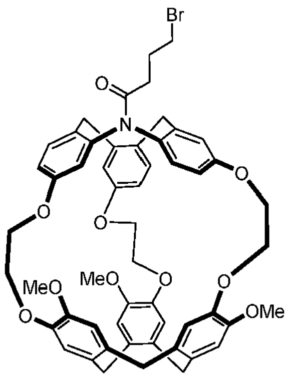
,
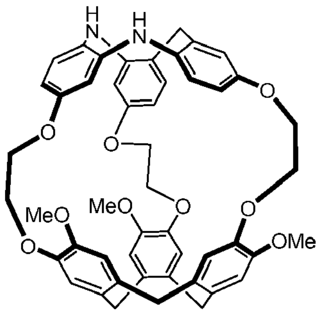
,
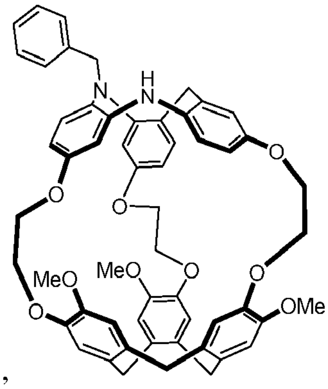
,
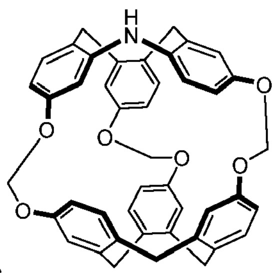
,
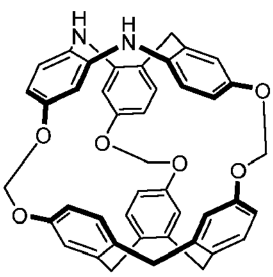
,
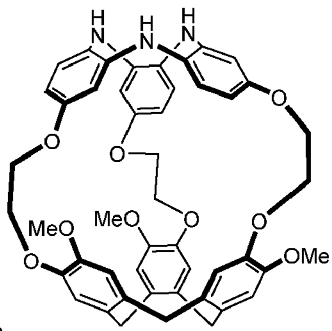
,
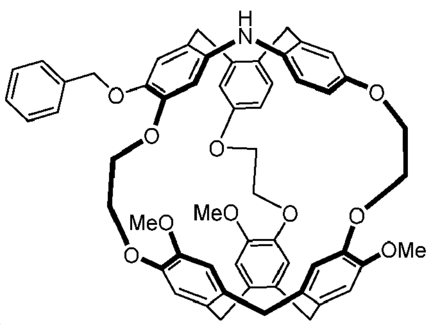
.

**7.** Process of preparation of a compound of formula (I) according to anyone of claims 1 to 6, wherein n1, n2 and n3 are independently chosen from 1, 2 and 3, comprising a step (i) of contacting a compound of following formula (II) with formic acid, $P_2O_5$, $HClO_4$, or $Sc(OTf)_3$:

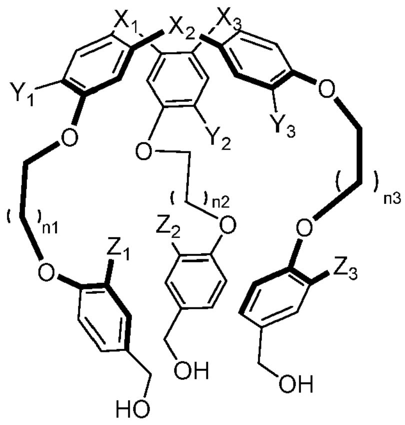

(II),

wherein :

$X_1$, $X_2$ and $X_3$ are independently selected from $-CH_2-$ and -NR-, at least one of $X_1$, $X_2$ and $X_3$ representing -NR-;
R is chosen from:

- H;
- a $C_1$-$C_6$ alkyl;
- a $C_2$-$C_6$ alkenyl;
- a $C_2$-$C_6$ alkynyl;
- an *ad hoc* protecting group, in particular a benzyl, *p*-methoxybenzyl, 2-tetrahydropyranyl, a carbamate, for example Boc, Fmoc, Troc, Cbz, a silyl, in particular *tert*-butyldimethylsilyl, trimethylsilyl, triisopropyl silyl, triethylsilyl, *tert*-butyldiphenylsilyl, or 2-(trimethylsilyl)ethoxymethyl, or an acyl group, in particular an acetyl;

$Y_1$, $Y_2$ and $Y_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- optionally a O-L'Z' group;
- a O-P' group, wherein P' is an *ad hoc* protecting group, in particular a benzyl; *p*-methoxybenzyl; silyl, in particular *tert*-butyldimethylsilyl, trimethylsilyl, triisopropylsilyl, triethylsilyl, *tert*-butyldiphenylsilyl ; 2-(trimethylsilyl)ethoxymethyl ; allyl; methoxymethyl ; tetrahydropyran or an acyl group, in particular an acetyl;

$Z_1$, $Z_2$ and $Z_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- optionally a O-L"Z" group;a O-P" group, wherein P" is an *ad hoc* protecting group, in particular a benzyl; *p*-methoxybenzyl; silyl, in particular *tert*-butyldimethylsilyl, trimethylsilyl, triisopropylsilyl, triethylsilyl, *tert*-butyldiphenylsilyl; 2-(trimethylsilyl)ethoxymethyl; allyl; methoxymethyl ; tetrahydropyran or an acyl group, in particular an acetyl;

L' and L" are independently from each other a linker;
Z' and Z" are independently from each other:

- a terminal reactive function or group, in particular able to form a covalent bond with a peptide, a protein, an antibody or a fragment thereof, a carrier, a solid support, a multivalent scaffold; an anchor; a dye or a fluorescent residue; or
- a peptide, a protein, an antibody or a fragment thereof, a carrier, a solid support, a multivalent scaffold; an anchor; a dye or a fluorescent residue;

n1, n2 and n3 are independently chosen from 0, 1, 2 and 3.

**8.** Process according to claim 7, wherein the compound of formula (II) is obtained by contacting a compound of following formula (III) with a compound of following formula (IV), in presence of a base, in particular a carbonate, more particularly $Cs_2CO_3$:

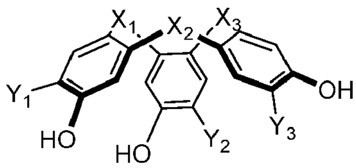
(III);
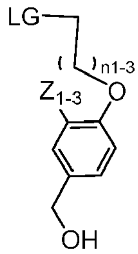
(IV),

wherein LG is a leaving group, in particular chosen from halogens, more particularly I, Br, Cl, notably I, and O-tosyl, the compound of formula (III) being in particular obtained by contacting a compound of following formula (V) with $BBr_3$:

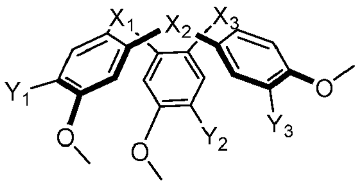
(V),

the compound of formula (V), when $X_1$ is $-CH_2-$ or -NR-, $X_2$ is -NR- and $X_3$ is $-CH_2-$, being more particularly obtained by contacting a compound of following formula (VI) with $HClO_4$:

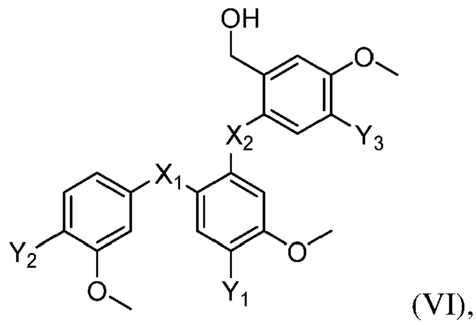
(VI),

wherein $X_1$ is $-CH_2-$ or -NR-, and $X_2$ is -NR-,
the compound of formula (V), when $X_1$ and $X_2$ are independently chosen from -NR- groups, R being not H, and $X_3$ is -NH-, being more particularly obtained from a compound of following formula (VII), in particular by Buchwald amination :

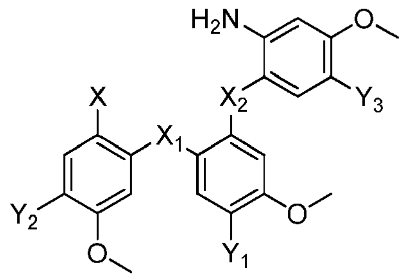

(VII),

wherein $X_1$ and $X_2$ are independently chosen from -NR- groups, R being not H, in particular - NP- groups, with P as defined above, and X is an halogen atom, in particular Br, I or Cl.

**9.** Process according to any one of claims 7 to 8:

- of a compound of formula (I) wherein $X_1$, $X_2$ and/or $X_3$, in particular $X_1$ and/or $X_2$, is - N(-LZ)-, said process comprising after step (i) a step of contacting a compound of formula (I) wherein $X_1$, $X_2$ and/or $X_3$, in particular $X_1$ and/or $X_2$, is -NH- with a compound of formula LG-LZ, wherein LG is a leaving group, LG being in particular a halogen, more particularly Cl, Br, I, F, preferably Br, or a O-tosyl group; or
- of a compound of formula (I) wherein $Y_1$, $Y_2$ and/or $Y_3$, in particular $Y_1$ and/or $Y_2$, is -O-L'Z', said process comprising after step (i) a deprotection step of a compound of formula (I) with $Y_1$, $Y_2$ and/or $Y_3$, in particular $Y_1$ and/or $Y_2$, being -OP, followed by the contacting the obtained compound of formula (I) wherein $Y_1$, $Y_2$ and/or $Y_3$, in particular Y1 and/or Y2, is -OH with a compound of formula LG'-L'Z', wherein LG' is a leaving group, LG' being in particular a halogen, more particularly Cl, Br, I, F, preferably Br, or a O-tosyl group;
- of a compound of formula (I) wherein $Z_1$, $Z_2$ and/or $Z_3$, in particular $Z_1$ and/or $Z_2$, is - O-L"Z", said process comprising after step (i):
- a deprotection step of a compound of formula (I) with $Z_1$, $Z_2$ and/or $Z_3$, in particular $Z_1$ and/or $Z_2$, being -OP", or
- a dealkylation step of a compound of formula (I) with $Z_1$, $Z_2$ and/or $Z_3$, in particular $Z_1$ and/or $Z_2$, being -O-$C_1$-$C_6$ alkyl, notably OMe,

followed by the contacting the obtained compound of formula (I) wherein $Z_1$, $Z_2$ and/or $Z_3$, in particular $Z_1$ and/or $Z_2$, is -OH with a compound of formula LG"-L" 'Z", wherein LG" is a leaving group, LG" being in particular a halogen, more particularly Cl, Br, I, F, preferably Br, or a O-tosyl group.

**10.** Process of preparation of a compound of formula (I) according to anyone of claims 1 to 6, wherein n1, n2 and n3 are 0, comprising:

- a step (i') of contacting a compound of formula (III) with a compound of following formula (VII), in presence of $BrCH_2Cl$ and a base, in particular $Cs_2CO_3$:

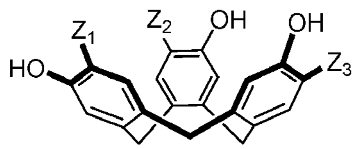

(VII),

Wherein $Z_1$, $Z_2$ and $Z_3$ are independently selected from:
- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- optionally a O-L"Z" group;
- a O-P" group, wherein P" is an *ad hoc* protecting group, in particular a benzyl; *p*-methoxybenzyl; silyl, in particular *tert*-butyldimethylsilyl, trimethylsilyl, triisopropylsilyl, triethylsilyl, *tert*-butyldiphenylsilyl ; 2-(trimethylsilyl)ethoxymethyl ; allyl; methoxymethyl ; tetrahydropyran or an acyl group, in particular an acetyl; or

- a step (i') of contacting a compound of formula (III), with $ClCH_2SMe$, then $SOCl_2$, and then with a compound of following formula (VII), in presence of a base, in particular $Cs_2CO_3$.

**11.** Compound of one of the following formulae:

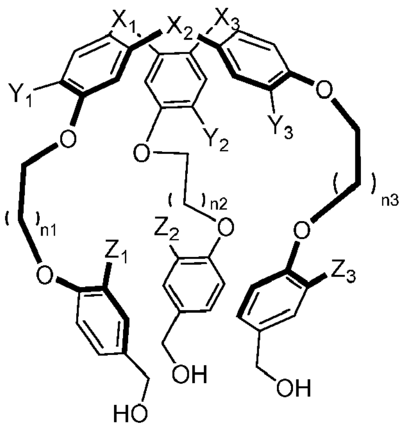
(II),
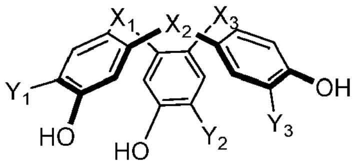
(III),

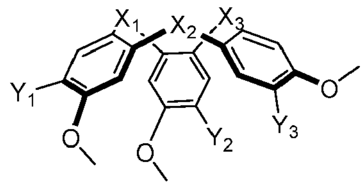
(V),

wherein :

$X_1$, $X_2$ and $X_3$ are independently selected from $-CH_2-$ and -NR-, at least one of $X_1$, $X_2$ and $X_3$ representing -NR-; R is chosen from:

- H;
- a $C_1$-$C_6$ alkyl;
- a $C_2$-$C_6$ alkenyl;
- a $C_2$-$C_6$ alkynyl;
- an *ad hoc* protecting group, in particular a benzyl, *p*-methoxybenzyl, 2-tetrahydropyranyl, a carbamate, for example Boc, Fmoc, Troc, Cbz, a silyl, in particular *tert*-butyldimethylsilyl, trimethylsilyl, triisopropyl silyl, triethylsilyl, *tert*-butyldiphenylsilyl, or 2-(trimethylsilyl)ethoxymethyl, or an acyl group, in particular an acetyl;

$Y_1$, $Y_2$ and $Y_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- optionally a O-L'Z' group;
- a O-P' group, wherein P' is an *ad hoc* protecting group, in particular a benzyl; *p*-methoxybenzyl; silyl, in particular *tert*-butyldimethylsilyl, trimethylsilyl, triisopropylsilyl, triethylsilyl, *tert*-butyldiphenylsilyl ; 2-(trimethylsilyl)ethoxymethyl ; allyl; methoxymethyl ; tetrahydropyran or an acyl group, in particular an acetyl;

$Z_1$, $Z_2$ and $Z_3$ are independently selected from:

- H,

- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- optionally a O-L"Z" group;
- a O-P" group, wherein P" is an *ad hoc* protecting group, in particular a benzyl; *p*-methoxybenzyl; silyl, in particular *tert*-butyldimethylsilyl, trimethylsilyl, triisopropylsilyl, triethylsily, *tert*-butyldiphenylsilyl ; 2-(trimethylsilyl)ethoxymethyl ; allyl; methoxymethyl ; tetrahydropyran or an acyl group, in particular an acetyl;

n1, n2 and n3 are independently chosen from 0, 1, 2 and 3.

**12.** Complex constituted of or comprising a compound of formula (I) according to anyone of claims 1 to 6 complexed with a hyperpolarized noble element, in particular Xe, more particularly $^{129}$Xe.

**13.** Compound of formula (I) according to anyone of claims 1 to 6 or complex according to claim 12 for its use as an in vivo diagnostic tool, in particular in NMR spectroscopy or in MRI applications.

**14.** Nanoemulsion oil-in-water comprising:

- An oil;
- An aqueous phase;
- Optionally, a surfactant;
- Optionally, a co-surfactant and/or a solubilizier; and
- A compound of following formula (Ia):

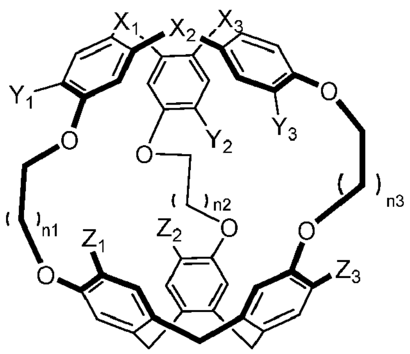

(Ia)

Wherein:

$X_1$, $X_2$ and $X_3$ are independently selected from $-CH_2-$ and -NR-, at least one of $X_1$, $X_2$ and $X_3$ representing -NR-;
R is chosen from:

- H;
- a $C_1$-$C_6$ alkyl;
- a $C_2$-$C_6$ alkenyl;
- a $C_2$-$C_6$ alkynyl;
- an *ad hoc* protecting group, in particular a benzyl, *p*-methoxybenzyl, 2-tetrahydropyranyl, a carbamate, for example Boc, Fmoc, Troc, Cbz, a silyl, in particular *tert*-butyldimethylsilyl, trimethylsilyl, triisopropylsilyl, triethylsilyl, *tert*-butyldiphenylsilyl, or 2-(trimethylsilyl)ethoxymethyl, or an acyl group, in particular an acetyl;

$Y_1$, $Y_2$ and $Y_3$ are independently selected from:

- H,

- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- OH;

$Z_1$, $Z_2$ and $Z_3$ are independently selected from:

- H,
- a $C_1$-$C_6$ alkyl;
- a $C_2$-$C_6$ alkenyl;
- a $C_2$-$C_6$ alkynyl;
- a O-$C_1$-$C_6$ alkyl;
- a O-$C_2$-$C_6$ alkenyl;
- OH;

n1, n2 and n3 are independently chosen from 0, 1, 2 and 3,
said compound of formula (I) being optionally complexed with a hyperpolarized noble element, in particular Xe, more particularly $^{129}Xe$.

**Figure 1**

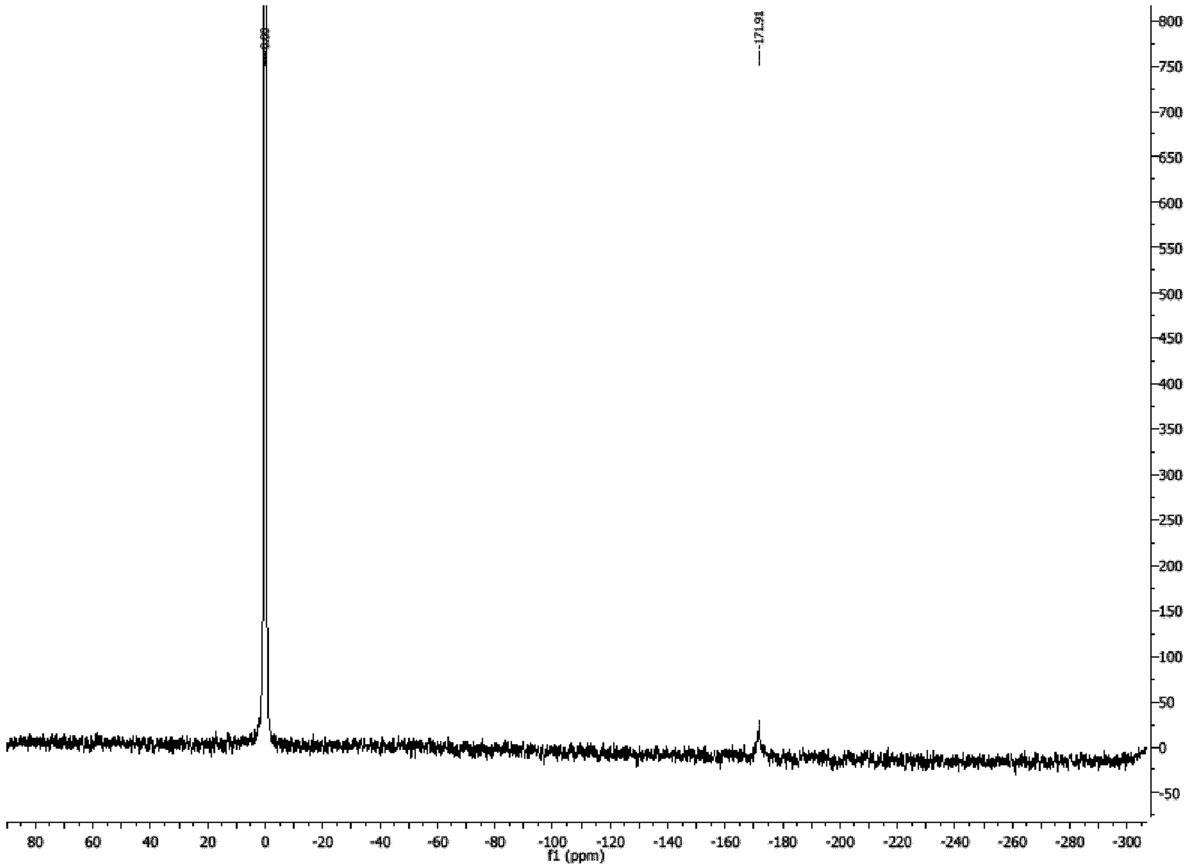

Figure 2

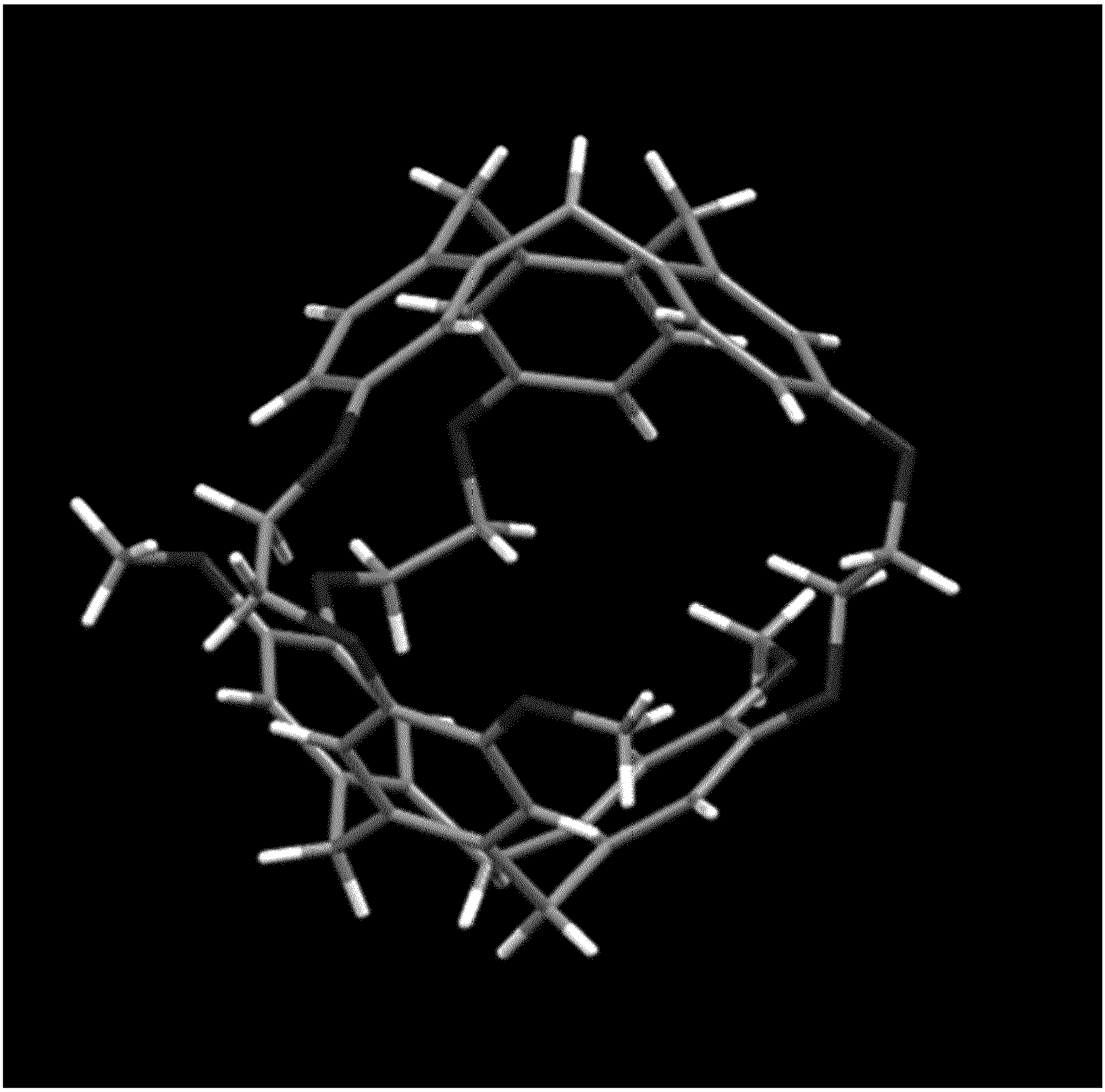

Europäisches Patentamt
European Patent Office
Office européen des brevets

# EUROPEAN SEARCH REPORT

Application Number
EP 21 30 5108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BERTHAULT PATRICK ET AL: "Sensitivity and Multiplexing Capabilities of MRI Based on Polarized 129 Xe Biosensors", JOURNAL OF THE AMERICAN CHEMICAL SOCIETY, vol. 130, no. 49, 14 November 2008 (2008-11-14), pages 16456-16457, XP055816717, ISSN: 0002-7863, DOI: 10.1021/ja805274u * the whole document * ----- | 1-14 | INV. C07D225/04 C07D245/04 C07D255/04 C07D498/22 A61K49/10 |
| Y | DELACOUR LÉA ET AL: ""Clickable" Hydrosoluble PEGylated Cryptophane as a Universal Platform for 129 Xe Magnetic Resonance Imaging Biosensors", CHEMISTRY - A EUROPEAN JOURNAL, vol. 19, no. 19, 13 March 2013 (2013-03-13), pages 6089-6093, XP055816734, ISSN: 0947-6539, DOI: 10.1002/chem.201204218 * the whole document * ----- | 1-14 | |
| | | | TECHNICAL FIELDS SEARCHED (IPC) |
| Y | STEFAN KLIPPEL ET AL: "Cell tracking with Caged Xenon: Using Cryptophanes as MRI Reporters upon Cellular Internalization", ANGEWANDTE CHEMIE INTERNATIONAL EDITION, VERLAG CHEMIE, DE, vol. 53, no. 2, 7 January 2014 (2014-01-07), pages 493-496, XP002737549, ISSN: 1433-7851, DOI: 10.1002/ANIE.201307290 [retrieved on 2013-12-04] * the whole document * ----- | 1-14 | C07D A61K |

-/--

The present search report has been drawn up for all claims

2

| Place of search | Date of completion of the search | Examiner |
|---|---|---|
| Munich | 23 June 2021 | Gavriliu, Daniela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 30 5108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MILANOLE GAËLLE ET AL: "Bimodal fluorescence/129Xe NMR probe for molecular imaging and biological inhibition of EGFR in Non-Small Cell Lung Cancer", BIOORGANIC & MEDICINAL CHEMISTRY, vol. 25, no. 24, 2017, - 2017, pages 6653-6660, XP085254606, ISSN: 0968-0896, DOI: 10.1016/J.BMC.2017.11.002 * the whole document * ----- | 1-14 | |
| Y | WO 2008/027162 A2 (UNIV PENNSYLVANIA [US]; DMOCHOWSKI IVAN [US] ET AL.) 6 March 2008 (2008-03-06) * claims; examples * ----- | 1-14 | |
| Y | US 2010/041880 A1 (BECKER DANIEL P [US] ET AL) 18 February 2010 (2010-02-18) * column 0064 - column 0068; claims; examples * ----- | 1-14 | |
| | | | TECHNICAL FIELDS SEARCHED (IPC) |

The present search report has been drawn up for all claims

2

| Place of search | Date of completion of the search | Examiner |
|---|---|---|
| Munich | 23 June 2021 | Gavriliu, Daniela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 21 30 5108

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2021

| Patent document cited in search report | Publication date | Patent family member(s) | Publication date |
|---|---|---|---|
| WO 2008027162 A2 | 06-03-2008 | US 2011104075 A1 | 05-05-2011 |
| | | WO 2008027162 A2 | 06-03-2008 |
| US 2010041880 A1 | 18-02-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ELIEL, E. L. ; WILEN, S.H.** Stereochemistry of Organic Compounds. Wiley, 1994
- **JACQUES, J et al.** Enantiomers, Racemates, and Resolutions. Wiley, 1981
- **R.C. LAROCK.** Comprehensive Organic Transformations. Wiley-VCH Publishers, 1999
- **T.W. GREENE ; P. G. M. WUTS.** Protective Groups in Organic Chemistry. John Wiley and Sons, 1999
- **J. F. W. MCOMIE.** Protective Groups in Organic Chemistry. Plenum Press, 1973
- **WALES et al.** *Org. Lett.,* 2019, vol. 21 (12), 4703-4708
- **SPENCE et al.** *J. Am. Chem. Soc.,* vol. 126 (46), 15287-15294
- **SPENCE et al.** *J. Am. Chem. Soc.,* 2004, vol. 126 (46), 15287-15294
- **XIE et al.** *Bioorganic Med. Chem.,* 2019, vol. 27 (13), 2764-2770
- **SÉGUY et al.** *Pharmaceutics.,* 2020, vol. 12, 1141